(19) Europäisches Patentamt
European Patent Office
Office européen des brevets

(11) **EP 3 956 904 B1**

(12) **EUROPEAN PATENT SPECIFICATION**

(45) Date of publication and mention
of the grant of the patent:
**09.04.2025 Bulletin 2025/15**

(21) Application number: **20717071.3**

(22) Date of filing: **16.04.2020**

(51) International Patent Classification (IPC):
*G16H 20/40* (2018.01)     *G16H 50/20* (2018.01)
*G16H 50/50* (2018.01)     *A61B 5/00* (2006.01)
*A61B 17/00* (2006.01)     *A61B 5/02* (2006.01)
*A61B 5/0215* (2006.01)

(52) Cooperative Patent Classification (CPC):
**G16H 20/40; A61B 5/02007; A61B 5/02158;
A61B 5/6852; G16H 50/20; G16H 50/50**

(86) International application number:
**PCT/EP2020/060642**

(87) International publication number:
**WO 2020/212459 (22.10.2020 Gazette 2020/43)**

(54) **MEANS AND DEVICES FOR ASSESSING CORONARY ARTERY DISEASE**

MITTEL UND VORRICHTUNGEN ZUR BEURTEILUNG DER KORONARARTERIENERKRANKUNG

MOYENS ET DISPOSITIFS POUR ÉVALUER LA CORONAROPATHIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.04.2019 GB 201905335**

(43) Date of publication of application:
**23.02.2022 Bulletin 2022/08**

(73) Proprietor: **ARTERIAX BV**
**1780 Wemmel (BE)**

(72) Inventors:
• **SONCK, Jeroen**
**1780 Wemmel (BE)**
• **COLLET BORTONE, Carlos Adolfo**
**1540 Herne (BE)**

(74) Representative: **De Clercq & Partners**
**Edgard Gevaertdreef 10a**
**9830 Sint-Martens-Latem (BE)**

(56) References cited:
US-A1- 2011 071 404     US-A1- 2014 207 008
US-A1- 2015 025 330     US-A1- 2016 008 084
US-A1- 2016 157 787     US-A1- 2016 157 802

**EP 3 956 904 B1**

**Description**

Field of the invention

The present invention relates to the field of cardiac disease, in particular to the assessment of coronary vessels, in particular to determine the mechanisms and patterns of blockage or restriction to the blood flow through a coronary vessel. The present invention provides diagnostic methods and devices to determine the condition of coronary artery disease, in specific to determine the functional pattern (focal or diffuse) of coronary artery disease.

Introduction to the invention

Physiological assessment of coronary artery disease has been encouraged since the early days of percutaneous coronary interventions. [1] In the last two decades, randomized controlled trials have confirmed the clinical benefit of invasive functional assessment to guide clinical decision making about myocardial revascularization in patients with stable coronary artery disease. [2, 3] In clinical practice, hemodynamic significance of epicardial coronary stenoses is assessed by means of pressure ratios. Fractional flow reserve (FFR), assessed as the pressure ratio between distal coronary and aortic pressure during pharmacologically-induced hyperaemia, depicts the maximal achievable flow in a coronary vessel. [4] FFR has been recommended by the American and European guidelines to determine lesion significance and appropriateness for revascularization. [5, 6] Treatment decision-making is based on one FFR value which provides a vessel level metric surrogate of myocardial ischemia. Pressure losses in the coronary arteries can ensue due to viscous friction and flow separation. The contribution of each of these components are depicted by the Bernoulli equation and Poiseuille Law and are highly dependent on patient-specific coronary geometries. Reduction in luminal area modulated by lesion length diminish pressure distal to epicardial stenosis. Also, lesion features affecting laminar flow conditions contribute to pressure drop. [7, 8] Along a normal coronary artery, no pressure loss is found even during maximal microvascular vasodilation [9]. In contrast, early stage coronary atherosclerosis is often associated with mild epicardial resistance in the coronary arteries before a segmental stenosis is apparent in invasive coronary angiography. This is identifiable by intra coronary pressure measurements and can contribute to the development of myocardial ischemia. [10]. Conventional coronary angiography has been traditionally used to assess stenosis significance and the spatial pattern of coronary artery disease (i.e. focal or diffuse). Nevertheless, coronary angiography is inaccurate in assessing the functional significance of a coronary stenosis when compared with the FFR. [11] Also, intravascular imaging and coronary computed tomography studies have revealed that coronary angiography underestimates the burden and the spatial distribution of coronary atherosclerosis. [12, 13] Moreover, using intravascular ultrasound, diffuse atherosclerosis is commonly observed in angiographically normal coronary artery reference segments in patients with stable coronary artery disease. [14] The relationship between atherosclerosis distribution its repercussion on luminal geometry and epicardial conductance along coronary vessels remains to be elucidated.

The distribution of epicardial conductance can be evaluated using an FFR pullback manoeuvre. [4] This technique reveals the contribution of focal and/or diffuse coronary artery disease (CAD) in terms of FFR drop along the coronary vessel.

A method for automated identification and classification of intravascular lesions is for example known from US2016/0157787. A method for assessing the severity of a blockage and simulating treatment options of a stenosis or lesion in a vessel is for example known from US2016/0008084. A method for automatically locating in an image the lumen boundary of a vessel to measure the diameter of the vessel, and based on the diameter of the vessel estimate blood flow rate to aid the clinician in the placement of a stent is for example known from US2011/071404.

The evaluation of the pattern of coronary artery disease (i.e. focal or diffuse) is one of the most compelling questions in interventional cardiology and accordingly there is a need for improved devices, systems and diagnostic methods for assessing the pattern of coronary artery disease. **It** is generally known that coronary vessels with diffuse pattern of coronary artery disease respond poorly to percutaneous coronary intervention with stent implantation. **In** contrast, vessels with focal disease respond favourably to percutaneous coronary intervention with stent implantation. **In** particular, there exists a need for diagnostic methods which can guide an interventional cardiologist with a treatment option in the different patterns of coronary artery disease.

Summary of the invention

According to a first aspect of the invention, there is provided a computer-implemented method for quantifying the patterns of coronary artery functional disease in a coronary vessel from a patient under hyperaemic conditions, comprising the following steps:

-    acquiring a set of relative pressure values obtained from:

- pressure values obtained at different positions along the coronary vessel between the ostium and the most distal part of the coronary vessel; relative to
- the pressure at the ostium of the vessel,

- mapping said set of relative pressure values along the coronary vessel length, and determining:

  - the contribution of the relative pressure drop of the functional disease with respect to the relative pressure drop over the total length of the coronary vessel; and
  - the extent of the functional disease, which corresponds to:

    - the length of suspected vessel lesions, with respect to the total length of the coronary vessel;
    - the length of suspected vessel lesions with relative pressure drops, with respect to the total length of the coronary vessel; or
    - the sum of the length of segments of the coronary vessel with relative pressure drops that are larger than or equal to a predetermined threshold, with respect to the total length of the coronary vessel.

According to an embodiment, there is provided a method, wherein the method comprises the further step of:

- calculating a functional outcome index (FOI) based on the combination of:

  - said contribution of the pressure drop of the functional disease to the pressure drop over the total length of the coronary vessel; and
  - said extent of the functional disease.

According to an embodiment, there is provided a method, wherein:

- said contribution of the pressure drop of the functional disease to the pressure drop over the total length of the coronary vessel corresponds to the ratio of:

  - the relative pressure drop between the proximal and distal edge of the functional disease, with respect to
  - the relative pressure drop between the ostium and the most distal end of the coronary vessel; and

- the extent of the functional disease, corresponds the ratio of

  - the length of the functional disease, with respect to
  - the total length of the coronary vessel;

According to an embodiment, there is provided a method, wherein:

- the length of the functional disease, corresponds to:

  - the length of suspected vessel lesions;
  - the length of suspected vessel lesions with relative pressure drops;
  - the sum of the length of segments of the coronary vessel with relative pressure drops that are larger than or equal to a predetermined threshold, with respect to the total length of the coronary vessel; or
  - the sum of the length of contiguous or non-contiguous segments of the coronary vessel with relative pressure drops that are larger than or equal to a predetermined threshold, and/or

- the extent of the functional disease, corresponds to:

  - the length of suspected vessel lesions, with respect to the total length of the coronary vessel;
  - the length of suspected vessel lesions with relative pressure drops, with respect to the total length of the coronary vessel;
  - the sum of the length of segments of the coronary vessel with relative pressure drops that are larger than or equal to a predetermined threshold, with respect to the total length of the coronary vessel, with respect to the total length of the coronary vessel; or
  - the sum of the length of contiguous or non-contiguous segments of the coronary vessel with pressure drops that are larger than or equal to a predetermined threshold, with respect to the total length of the coronary vessel.

According to an embodiment, there is provided a method, wherein the predetermined threshold is equal to a relative pressure drop of 0.0015 per mm of length of the coronary vessel.

According to an embodiment, there is provided a method, wherein the method comprises the steps of:

- acquiring a fractional flow reserve (FFR) pullback curve based on a multiple of FFR values obtained at different positions of the coronary vessel between the ostium and the most distal part of the coronary vessel,
- mapping said multiple of FFR values along the coronary vessel length, and determining:

  - the contribution of said FFR drop of the functional disease with respect to the FFR drop over the total length of the coronary vessel; and
  - said extent of the functional disease.

According to an embodiment, there is provided a method, wherein the method comprises said step of:

- calculating said functional outcome index (FOI) on the data from the FFR pullback curve, such that the FOI is an expression of at least one of the following functional patterns of coronary artery disease:

  - a focal functional coronary artery disease;
  - a diffuse functional coronary artery disease.

According to an embodiment, there is provided a method, wherein the method comprises said step of:

- calculating said functional outcome index (FOI) on the data from the FFR curve based on formula:

$$FOI = \frac{\frac{\Delta\,FFR\,lesion}{\Delta\,FFR\,vessel} + \left(1 - \left(\frac{Length\;with\;FFR\;drop}{Total\;vessel\;length}\right)\right)}{2}$$

wherein $\Delta FFR_{lesion}$ is defined as the difference between FFR values at the proximal and distal edge of the functional disease; $\Delta FFR_{vessel}$ as the difference between FFR values between the ostium and the most distal part of the coronary vessel; Length with FFR drop is defined as the sum of contiguous millimeters with FFR drop $\geq 0.0015$; and the total vessel length is the distance between the ostium and the most distant part of the coronary vessel.

According to an embodiment, there is provided a method, wherein, when the value of the FOI:

- is higher than 0.7, this indicates the functional pattern of a focal coronary artery disease; and/or
- is lower than 0.4, this indicates the functional pattern of a diffuse coronary artery disease.

According to an embodiment, there is provided a method, wherein said set of multiple of relative pressure values were obtained:

- by means of a manual or motorized pullback of a pressure wire comprising at least one pressure sensor;
- by means of a pressure wire comprising a multiple of built-in pressure sensors;
- from Angiography-derived FFR values along the length of the coronary vessel; and/or
- from CT Angiography-derived FFR values along the length of the coronary vessel.

According to a second aspect, there is provided a computer device for evaluating coronary artery disease in a patient under hyperaemic conditions, said computer device configured to generate an FFR curve based on a multiple of FFR values, which are relative pressure measurements from pressures obtained at different positions along the total length of the coronary vessel between the ostium and the most distal part of the coronary vessel, relative to the pressure at the ostium of the coronary vessel, and wherein said computer device is further configured to map said multiple of FFR values along the coronary vessel length, and to determine:

- the contribution of said FFR drop of the functional disease with respect to the FFR drop over the total length of the coronary vessel; and
- said extent of the functional disease, which corresponds to:

- the length of suspected vessel lesions, with respect to the total length of the coronary vessel;
- the length of suspected vessel lesions with relative pressure drops, with respect to the total length of the coronary vessel; or
- the sum of the length of segments of the coronary vessel with relative pressure drops that are larger than or equal to a predetermined threshold, with respect to the total length of the coronary vessel.

According to an embodiment, there is provided a computer device, wherein: said computer device comprises a computer algorithm configured to calculate a functional outcome index (FOI) based on the combination of:

- said contribution of the pressure drop of the functional disease to the pressure drop over the total length of the coronary vessel; and
- said extent of the functional disease.

According to an embodiment, there is provided a computer device, wherein said computer device comprises a computer algorithm configured to calculate a functional outcome index (FOI) based on the FFR curve and the correlation of the FFR values over the total length of the vessel, the computer output configured to display an FOI value, such that the FOI value is an expression of at least one of the following functional patterns of coronary artery disease:

- a focal functional coronary artery disease;
- a diffuse functional coronary artery disease.

According to an embodiment, there is provided a computer device, wherein said computer device comprises a computer algorithm configured to calculate said functional outcome index (FOI) on the data from the FFR curve based on formula:

$$FOI = \frac{\frac{\Delta\,FFR\ lesion}{\Delta\,FFR\ vessel} + \left(1 - \left(\frac{Length\ with\ FFR\ drop}{Total\ vessel\ length}\right)\right)}{2}$$

wherein $\Delta FFR_{lesion}$ is defined as the difference between FFR values at the proximal and distal edge of the functional disease; $\Delta FFR_{vessel}$ as the difference between FFR values between the ostium and the most distal part of the coronary vessel; Length with FFR drop is defined as the sum of contiguous millimeters with FFR drop $\geq 0.0015$; and the total vessel length is the distance between the ostium and the most distant part of the coronary vessel.

According to an embodiment, there is provided a computer device further configured to co-register the relative pressure measurements with the positions in the coronary vessel. According to a particular embodiment the co-registration of the position in the coronary vessel could be performed by means of angiography, however according to alternative embodiments, the position embodiment could be derived from a measurement and/or registration of the displacement of the pressure wire with respect to the catheter, for example by means of a suitable sensor configured to determine the displacement and or distance by the pressure wire and the at least one sensor thereon with respect to the catheter, such as any suitable position sensor, such as for example a linear position sensor, an opto-electronic displacement sensor, etc. or according to sill further alternative embodiments by means of a suitable visual scale present on the pressure wire, that allows for a manual input of the displacement and/or the relative position of the pressure wire with respect to the catheter, or in other words, how far or over what length the pressure wire and its corresponding at least one sensor are introduced into the vessel with respect to the ostium of the vessel.

According to an embodiment, there is provided a system, wherein the system comprises at least one of the following, in communication with the computer device, and configured to generate the multiple FFR values:

- A catheter and a pressure wire comprising at least one pressure sensor,
- A catheter and a pressure wire coupled to a motorized device with a fixed pullback speed;
- A catheter and a pressure wire comprising a multiple of built-in pressure sensors;
- A device configured to provide Angiography-derived FFR values along the length of the coronary vessel;

- A device configured to provide CT Angiography-derived FFR values along the length of the coronary vessel

Further embodiments of the system, in which the computer device implements the embodiments of the computer-implemented method according to the first aspect and/or combinations thereof are possible.

According to a further aspect there is provided a method for quantifying the patterns of coronary artery functional disease in a coronary vessel from a patient comprising the following steps:

- obtaining pressure values at different positions along the coronary vessel between the ostium and the most distal part of the coronary vessel; and the pressure at the ostium of the vessel;
- generating a set of relative pressure values by means of:

  - the pressure values obtained at different positions along the coronary vessel between the ostium and the most distal part of the coronary vessel; relative to
  - the pressure at the ostium of the vessel,

- mapping said set of relative pressure values along the coronary vessel length, and determining:

  - the contribution of the relative pressure drop of the functional disease with respect to the relative pressure drop over the total length of the coronary vessel; and
  - the extent of the functional disease.

Further embodiments of the method, in which the method implements the embodiments of the computer-implemented method according to the first aspect and/or combinations thereof are possible.

Further, there is provided, according to a further aspect, a method, further comprising the step of informing an interventional cardiologist with a treatment option for the coronary vessel based on the value of the FOI, wherein:

- when the value of the FOI is higher than 0.7, this indicates the presence of a focal lesion in the coronary vessel, and/or treatment with percutaneous coronary intervention with stent implantation should be considered;
- when the value of FOI is in the range of 0.5 to 0.7, this indicates the presence of a combination of focal and/or diffuse lesions, and/or treatment with percutaneous coronary intervention with stent implantation might still be considered; and/or
- when the FOI is less than 0.4, this indicates the presence of diffuse lesions, and/or treatments other than percutaneous coronary intervention with stent implantation should be considered, or treatment with percutaneous coronary intervention with stent implementation should not be considered.

According to an embodiment we characterized in a systematic manner the physiological patterns of coronary artery disease (CAD) using manual or motorized coronary pressure pullbacks during continuous hyperaemia in patients with stable coronary artery disease. Standardization of the pressure-length relationship of the coronary artery was accomplished by motorising FFR pullbacks which allowed for accurate and reproducible tracings. We developed a new algorithm which calculates the functional outcomes index (FOI). This new parameter is based on the functional impact of anatomical lesions on coronary artery disease. In still other words, the FOI is a continuous metric wherein values approaching "1.0" represent focal physiological coronary artery disease and values close to "0" represent diffuse CAD. The FOI value has therefore a direct impact on the treatment decision for an interventional cardiologist. According to such an embodiment the functional outcome index (FOI) is calculated on the data from the FFR pullback curve based on formula:

$$ FOI = \frac{\frac{\Delta\,FFR\;lesion}{\Delta\,FFR\;vessel} + \left(1 - \left(\frac{Length\;with\;FFR\;drop}{Total\;vessel\;length}\right)\right)}{2} $$

wherein $\Delta FFR_{lesion}$ is defined as the difference between FFR values at the proximal and distal edge of the functional disease; $\Delta FFR_{vessel}$ as the difference between FFR values between the ostium and the most distal part of the coronary vessel; Length with FFR drop is defined as the sum of contiguous millimeters with FFR drop $\geq 0.0015$; and the total vessel length is the distance between the ostium and the most distant part of the coronary vessel. It is clear that in this way the ratio of $\frac{\Delta\,FFR\;lesion}{\Delta\,FFR\;vessel}$ varies between 0 and 1 or in other words 0% and 100%. It is further also clear that the extent of the functional disease of the coronary vessel as defined by the ratio $\frac{Length\;with\;FFR\;drop}{Total\;vessel\;length}$ also varies between 0 and 1 or in other words 0% and 100%. It is thus clear that in the formula above both terms of the sum combine to a value that varies between 0 and 2, and that thus a division by 2 can be performed in order to arrive at a value for FOI which varies between 0 and 1 or in other words 0% and 100%.

According to a further aspect there is provided a diagnostic method for quantifying artery disease in a coronary vessel from a patient comprising the following steps:

i) generating a fractional flow reserve (FFR) pullback curve based on a multiple of FFR values obtained between the ostium of the vessel and the most distal part of the vessel,
ii) calculating a functional outcome index (FOI) on the data from the FFR curve based on formula (I):

$$FOI = \frac{\Delta\, FFR\ lesion}{\Delta\, FFR\ vessel} + \left(1 - \left(\frac{Length\ with\ FFR\ drop}{Total\ vessel\ length}\right)\right)$$

(I)

wherein $\Delta FFR_{lesion}$ is defined as the difference between FFR values at the proximal and distal lesion edge of the lesion; $\Delta FFR_{vessel}$ as the difference between FFR values between the ostium of the vessel and the most distal FFR measurement in the vessel; length with FFR drop is defined as the sum of contiguous millimeters with FFR drop $\geq$ 0.0015; and the total vessel length is the distance between the ostium and the most distant part of the vessel.

According to an embodiment, there is provided a diagnostic method, wherein the multiple of FFR values were obtained by a motorized pullback.
According to an embodiment, there is provided a diagnostic method, wherein the multiple of FFR values were obtained by a pressure wire comprising a multiple of built-in pressure sensors.
According to an embodiment, there is provided a diagnostic method, further comprising informing an interventional cardiologist with a treatment option for the coronary vessel based on the value of the FOI, wherein when the value of the FOI is higher than 0.7 indicates the presence of a focal lesion in the coronary vessel.
According to an embodiment, there is provided a diagnostic method, which suggests the interventional cardiologist no intervention or an intervention wherein an intervention comprises an angioplasty, a stent, a pharmaceutical or a combination thereof.
According to a further aspect, there is provided a system for evaluating coronary artery disease in a patient under hyperaemic conditions, comprising

i) a coronary catheter comprising a pressure sensor, said catheter further comprising a pressure wire comprising at least one pressure sensor,
ii) a computing device in communication with the catheter and the pressure wire, the computing device configured to generate an FFR curve based on a multiple of FFR values, which are relative pressure measurements from pressures obtained over the total length of the coronary vessel relative to the pressure in the ostium,
iii) said computer device comprising a computer algorithm which calculates a functional outcome index (FOI) based on the FFR pullback curve and the correlation of the FFR values obtained in step ii) over the total length of the vessel, the computer output displays an FOI value which informs an interventional cardiologist of a treatment option based on the likelihood for the presence of focal or diffuse coronary artery disease in the coronary artery.

According to an embodiment, there is provided a system, wherein the pressure wire said pressure wire is coupled to a motorized device with a fixed pullback speed.

Figures

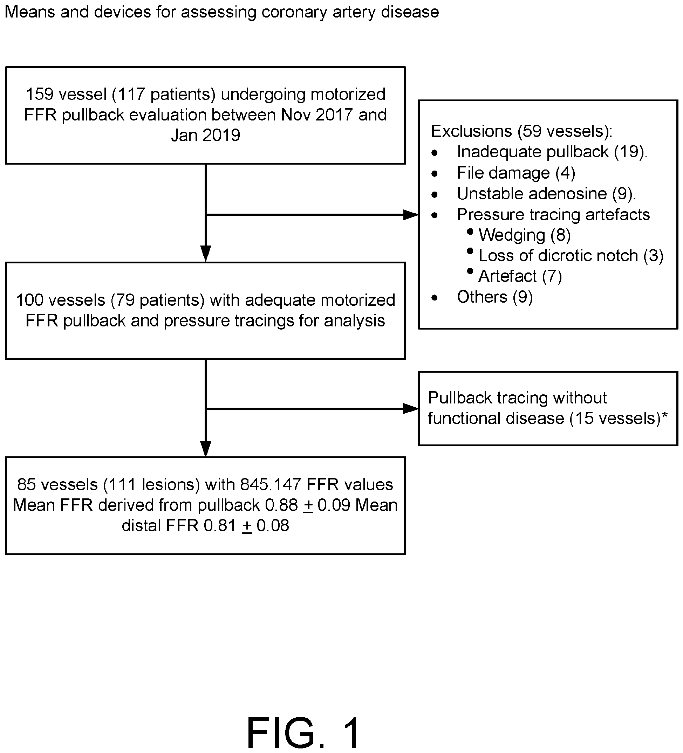

Figure 1: Flowchart of the patients included in the study.

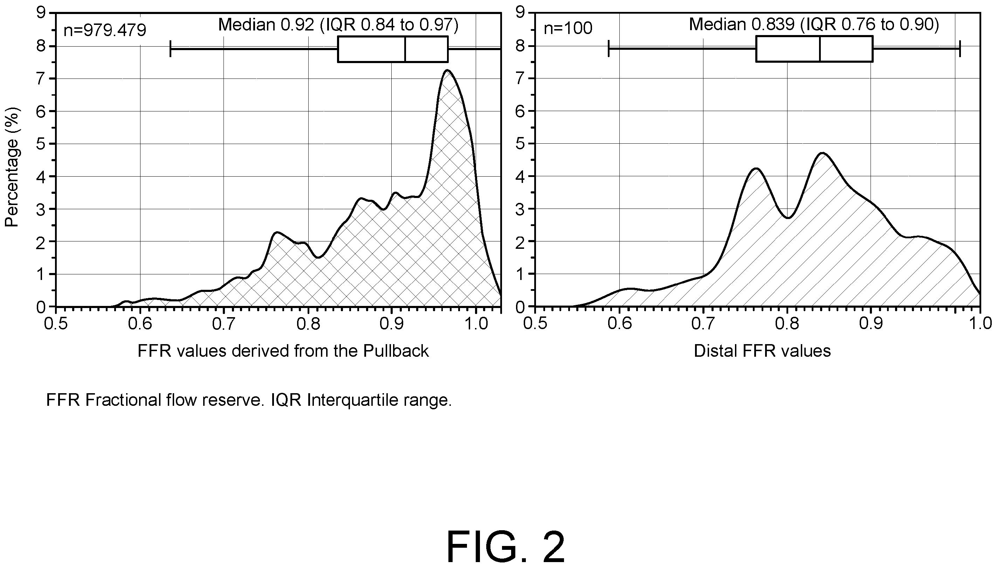

Figure 2: Distribution of FFR values derived from the pullbacks and at the distal vessel position. The left panel shows the distribution of FFR values derived from the motorized pullbacks. The right panel depicts the distribution of distal FFR values.

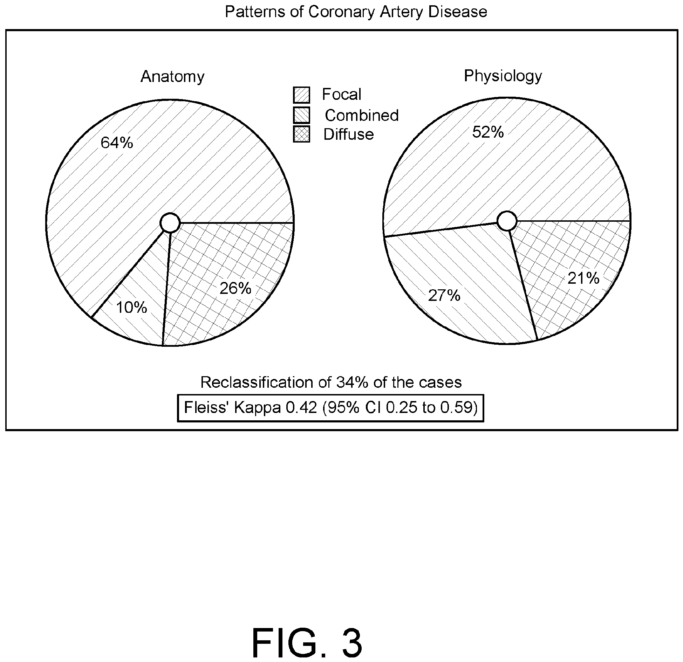

Figure 3: Reclassification between anatomical and physiological assessment on the pattern of coronary artery disease. The left pie chart presents the classification of the pattern and CAD based on coronary angiography (n=85 vessels). The pie chart on the right shows de classification of the CAD patterns assessed using the motorized FFR pullback curve.

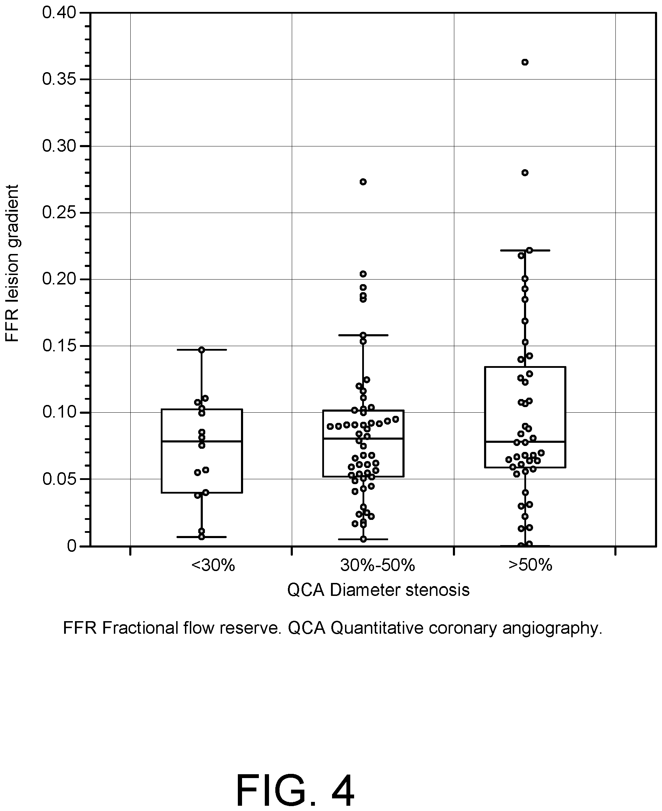

Figure 4: Fractional flow reserve lesion gradient and percent diameter stenosis. FFR lesion gradients stratified according the anatomical severity of CAD measured by percent diameter stenosis. No significant differences were observed concerning lesion FFR gradient between lesions with <30% percent diameter stenosis, 30% to 50% or more than 50%.

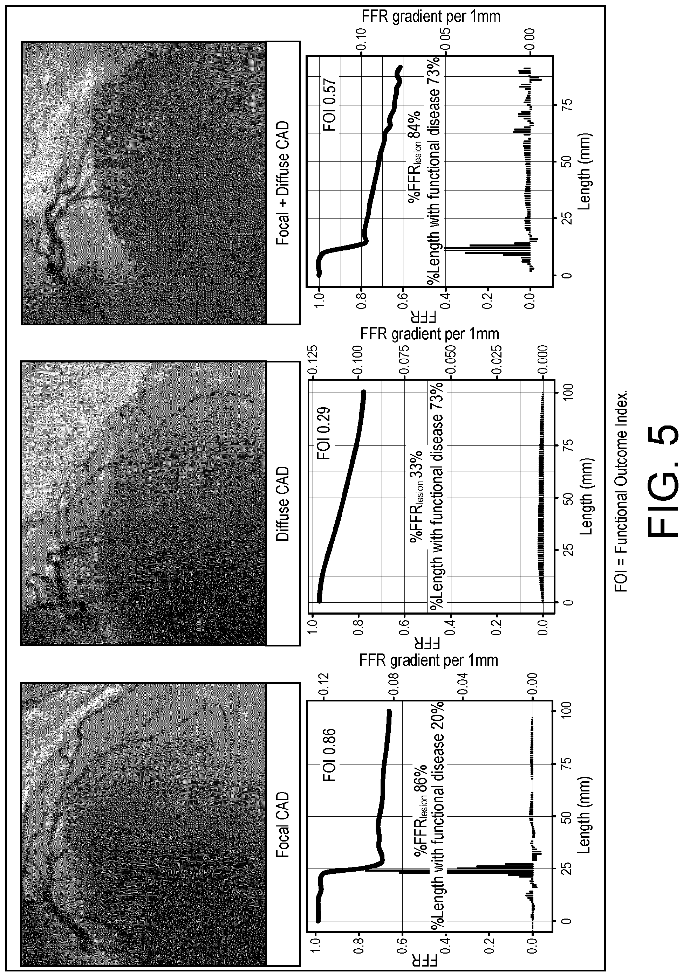

Figure 5: Case examples of physiological coronary artery disease patterns and functional outcomes index (FOI). Three case examples depicting the physiological patterns of CAD. On the left panel, the angiography shows a severe lesion in the mid LAD (white star) with a distal FFR of 0.68. This lesion produced an FFR drop responsible for 86% of the distal FFR. Only 20% of the vessel showed physiological disease. The FOI was 0.86 indicating physiological focal CAD. The mid panel shows an anatomical lesion in the mid LAD (white star) with distal FFR of 0.78. This lesion was responsible for 33% of the vessel FFR drop while 73% of the vessel showed to have physiological disease. The FOI was 0.29, indicating physiological diffuse CAD. In the right panel a severe lesion in the ostial LAD (white star) is observed, the distal FFR was 0.62. This lesion was responsible for 84% of the vessel FFR. However, in the proximal and mid LAD mild stenoses create a diffuse pressure drop that also contribute to the vessel FFR. The FOI was 0.57. LAD Left anterior descending artery. CAD Coronary artery disease. FFR Fractional flow reserve. FOI Functional outcome index.

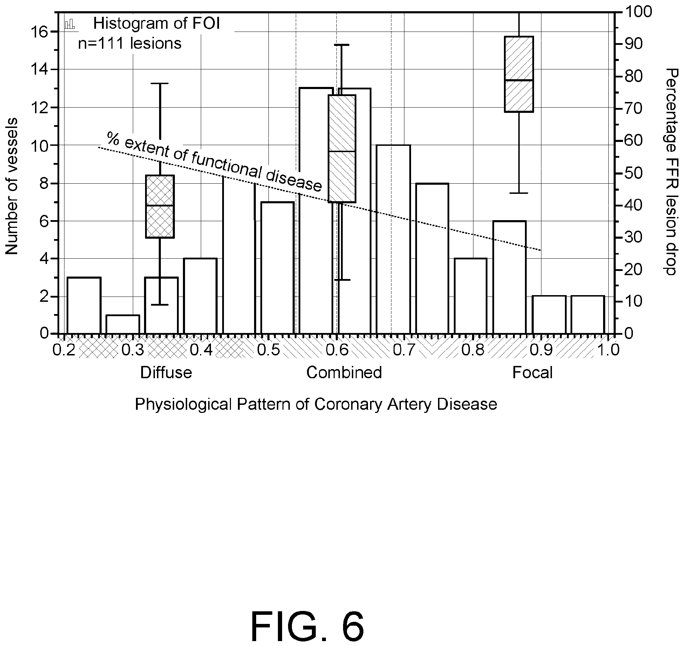

Figure 6: Distribution of the functional outcomes index. The grey bars show the distribution of the functional outcomes index (FOI), the number of vessels is shown in the left y-axis. The box plots show the median lesion FFR gradient divided by vessel FFR gradient ($\%FFR_{lesion}$) stratified by FOI tertiles (dashed blue lines). The $\%FFR_{lesion}$ was significantly different between tertiles (p<0.001). The percent extent of the vessel with functional disease is shown by the black dashed line. The mean value is plotted for each FOI tertile and was significantly different between the FOI tertiles (p<0.001). The right y-axis denotes percentage for the $\%FFR_{lesion}$ and % extent of physiological disease.

### Detailed description of the invention

The present invention will be described with respect to particular embodiments and with reference to certain drawings, but the invention is not limited thereto but only by the claims. Any reference signs in the claims shall not be construed as limiting the scope. The drawings described are only schematic and are nonlimiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. The following terms or definitions are provided solely to aid in the understanding of the invention. Unless specifically defined herein, all terms used herein have the same meaning as they would to one skilled in the art of the present invention. Unless defined otherwise, all technical and scientific terms used herein have the same meaning as commonly understood by one of ordinary skill in the art (e.g. in molecular biology, interventional cardiology fluid physics, biochemistry, and/or computational biology).

Randomized controlled trials have confirmed the clinical benefit of invasive functional assessment to guide clinical decision making about myocardial revascularization in patients with stable coronary artery disease. Treatment decision is currently based on only one FFR value which provides a vessel level metric surrogate of myocardial ischemia. In the present invention we characterized the physiological patterns of coronary artery disease using manual or motorized coronary pressure pullbacks during continuous hyperaemia in patients with stable coronary artery disease. In our prospective, multicentre study of patients undergoing clinically-indicated coronary angiography, a pullback device, adapted to grip the coronary pressure wire was set at a speed of 1 mm/sec. The pattern of coronary artery disease was adjudicated based on coronary angiography and on the manual or motorized FFR pullback curve as focal, diffuse or as a combination of both mechanisms. Also, a quantitative assessment of the physiological pattern of coronary artery disease was established by computing the functional outcomes index (FOI). The FOI is a continuous metric, values approaching 1.0 represent focal physiological CAD and value close to 0 diffuse CAD.

Thus, the present invention provides a new diagnostic method which incorporates a new metric, the functional outcome index (FOI). The FOI takes into account the functional impact of anatomical lesions and the extent of physiological disease, and the FOI value differentiates focal from diffuse CAD.

Accordingly, the present invention provides in a first embodiment a method for assessing a treatment option for a lesion present in a coronary vessel during continuous infusion of a hyperemic agent comprising the following steps:

i) introducing a coronary catheter comprising a pressure sensor into the ostium of the left or right coronary vessel followed by the introduction of a guide wire comprising at least one built-in pressure sensor,

ii) acquiring a set of relative pressure values obtained from pressure values obtained at different positions along the coronary vessel relative to the pressure present at the fixed position of the coronary catheter,

iii) mapping said set of relative pressure values along the coronary vessel length and determining the length of the vessel and the length of suspected vessel lesions or in other words the length of the functional disease. It is clear that the extent of the functional disease thus corresponds to the ratio of the length of the functional disease with respect to the length of the total vessel.

iv) optionally correlating the values obtained in step iii) with a quantitative coronary angiography,

v) calculating the functional outcome index (FOI) based on a combination of:

- the proportion of coronary pressure dropped in the suspected lesions relative to the pressure drop in the full vessel; and

- the extent of the functional coronary artery disease, and

- wherein the FOI is an expression, of the functional pattern of the coronary artery disease,

vi) displaying the results of the FOI to aid in a treatment decision for revascularization for at least one lesion present in the coronary vessel.

It is clear that the pressure dropped or relative pressure dropped in the suspected lesions, corresponds to the aggregation or sum of pressure drop or relative pressure drop at the location of all the suspected lesions along the coronary vessel. In other words, the pressure drop and/or relative pressure drop between the proximal and distal edge in case of a single, continuous suspected lesion, or, in case of multiple, serial and/or discontinuous suspected lesions the sum or aggregation of the pressure drop and/or relative pressure drops for each lesion between its respective proximal and distal end. Or in other words, it is clear that the relative pressure drop between the proximal and distal edge of the functional disease corresponds to difference between the relative pressure value at the distal end of the functional disease and the relative pressure value at the proximal end of the functional disease. It is further clear that according to particular embodiments the FFR drop of the functional disease, or the FFR drop of the suspected lesions corresponds to the FFR at the distal end of the functional disease or suspected lesions, minus the FFR at the proximal end of the functional disease or suspected lesions. Similarly it is clear that the relative pressure drop between the ostium and the most distal end of the coronary vessel, corresponds to the difference, delta or gradient between the most distal relative pressure measurement of the vessel and the ostial relative pressure measurement of the vessel. According to particular embodiments this thus means the difference between the FFR at the distal end of the vessel and the FFR at the ostium of the vessel.

In yet another embodiment the invention provides a method for assessing a treatment option for a lesion present in a coronary vessel during and/or after infusion, such as for example continuous infusion or any other suitable type of infusion, of a hyperemic agent comprising the following steps:

i) introducing a coronary catheter comprising a pressure sensor into the ostium of the left or right coronary vessel followed by the introduction of a guide wire comprising at least one built-in pressure sensor,

ii) acquiring a set of relative pressure values obtained from pressure values obtained at different positions along the coronary vessel relative to the pressure present at the fixed position of the coronary catheter,

iii) mapping said set of relative pressure values along the coronary vessel length and determining the length of the vessel and the length of suspected vessel lesions,

iv) optionally correlating the values obtained in step iii) with a quantitative coronary angiography,

v) calculating the functional outcome index (FOI) based on: the proportion of coronary pressure dropped in the suspected lesions relative to the pressure drop in the full vessel; and the extent of the suspected lesions, or in other words the extent of the functional disease, and wherein the FOI is an expression of the functional pattern coronary artery disease, or calculating the functional outcome index (FOI) based on: the proportion of coronary pressure dropped in the suspected lesions relative to the pressure drop in the full vessel; and the extent of functional coronary artery disease,

vi) displaying the results of the FOI to aid in a treatment decision for revascularization for at least one lesion present in the coronary vessel,

vii) wherein when the FOI is less than 0.4 treatments other than percutaneous coronary intervention with stent implantation should be considered.

It is thus clear that according to an embodiment, when the FOI is lower than 0.4, this indicates the functional pattern of a diffuse coronary artery disease. It is however clear that alternative embodiments are possible in which, for example a functional pattern of a diffuse coronary artery disease is indicated when the FOI is lower than a suitable maximum threshold, such as for example lower than 0.3, lower than 0.2, or lower than 0.15

The wording 'the pressure drop in the full vessel' means the pressure difference obtained between the pressure measured at the ostium of the coronary vessel and the pressure obtained at the most distal part of the coronary vessel.

In yet another embodiment the invention provides a method for assessing a treatment option for a lesion present in a coronary vessel under hyperaemic conditions, for example upon a bolus injection or during continuous infusion of a hyperemic agent, comprising the following steps:

i) introducing a coronary catheter comprising a pressure sensor into the ostium of the left or right coronary vessel followed by the introduction of a guide wire comprising at least one built-in pressure sensor,

ii) acquiring a set of relative pressure values obtained from pressure values obtained at different positions along the coronary vessel relative to the pressure present at the fixed position of the coronary catheter, using a motorized distal device with fixed pullback speed,

iii) mapping said set of relative pressure values along the coronary vessel length and determining the length of the vessel and the length of suspected vessel lesions,

iv) optionally correlating the values obtained in step iii) with a quantitative coronary angiography,

v) calculating the functional outcome index (FOI) based on the combination of: the proportion of coronary pressure dropped in the suspected lesions relative to the pressure drop in the full vessel and the extent of the suspected lesions; and wherein the FOI is an expression of the functional pattern coronary artery disease, or calculating the functional outcome index (FOI) which is based on the combination of: the proportion of coronary pressure dropped in the suspected lesions relative to the pressure drop in the full vessel; and the extent of functional coronary artery disease,

vi) displaying the results of the FOI to aid in a treatment decision for revascularization for at least one lesion present in the coronary vessel,

It is thus clear that the length of the vessel, also referred to as the total vessel length, can be determined by determining the distance between and/or the difference between the positions mapped to the pressure values associated with the ostium and the most distal part of the coronary vessel.

In yet another embodiment the invention provides a diagnostic method for quantifying artery disease in a coronary vessel from a patient comprising the following steps:

i) generating an FFR curve based on a multiple of FFR values obtained between the ostium of the vessel and the most distal part of the vessel,

ii) calculating a functional outcome index (FOI) on the data from the FFR curve based on formula:

$$FOI = \frac{\dfrac{\Delta\,FFR\,lesion}{\Delta\,FFR\,vessel} + \left(1 - \left(\dfrac{Length\,with\,FFR\,drop}{Total\,vessel\,length}\right)\right)}{2}$$

wherein $\Delta FFR_{lesion}$ is defined as the difference between FFR values at the proximal and distal lesion edge of the lesion; $\Delta FFR_{vessel}$ as the difference between FFR values between the ostium of the vessel and the most distal FFR measurement in the vessel; length with FFR drop is defined as the sum of contiguous millimeters with FFR drop $\geq$ 0.0015; and the total vessel length is the distance between the ostium and the most distant part of the vessel.

In the present invention the terms a 'guide wire comprising at least one pressure sensor' or a 'pressure wire' are equivalent.

In a specific embodiment the fractional flow reserve (FFR) curve is obtained by a manual or motorized pullback device which device is attached to the pressure wire.

In yet another particular embodiment there is no need for a motorized pullback device but instead the FFR curve is obtained by a pressure wire comprise a multiple of built-in pressure sensor. Particularly, the FOI value does not change when the pullback is carried out manually or with the aid of a motorized device. In particular embodiments the diagnostic methods of the invention provide a treatment suggestion to an interventional cardiologist based on the value of the FOI, wherein when the value of the FOI is higher than 0.7, higher than 0.8 or higher than 0.9 indicates the presence of a focal lesion in the coronary vessel and benefits from percutaneous coronary intervention with stent implantation. In particular embodiments the diagnostic methods of the invention provide a treatment suggestion to an interventional cardiologist based on the value of the FOI, wherein when the value of the FOI is preferably lower than 0.4, lower than 0.3 or lower than 0.2, lower than 0.15, this indicates the presence of a diffuse lesion in the coronary vessel and does not benefit from percutaneous coronary intervention with stent implantation. It has further been found that a treatment suggestion to an interventional cardiologist based on the value of the FOI, wherein when the value of the FOI is higher than 0.4 and lower than 0.7, such as for example in the range of 0.5 to 0.7 which indicates the presence of a combination of focal and diffuse lesions in the coronary vessel, can be made, that there might still be a benefit from percutaneous coronary intervention with stent implantation. However, with an FOI lower than 0.4 there will be no benefit from does not benefit from a treatment with percutaneous coronary intervention with stent implantation.

In a particular embodiment, the catheter is configured to obtain diagnostic information about the coronary vessel. In this respect, the catheter can include one or more sensors, transducers, and/or other monitoring elements configured to obtain the diagnostic information about the vessel. The diagnostic information includes one or more of pressure, flow (velocity), images (including images obtained using ultrasound (e.g. IVUS), optical coherence tomography (OCT), thermal, and/or other imaging techniques), temperature, and/or combinations thereof. These one or more sensors, transducers, and/or other monitoring elements are positioned less than 30 cm, less than 10 cm, less than 5 cm, less than 3 cm, less than 2 cm, and/or less than 1 cm from a distal tip of the catheter in some instances. In some instances, at least one of the one or more sensors, transducers, and/or other monitoring elements is positioned at the distal tip of the catheter. In another particular embodiment the catheter comprises at least one element configured to monitor pressure within the coronary vessel. The pressure monitoring element can take the form a piezo-resistive pressure sensor, a piezo-electric pressure sensor, a capacitive pressure sensor, an electromagnetic pressure sensor, an optical pressure sensor, and/or combinations thereof. In some instances, one or more features of the pressure monitoring element are implemented as a solid-state component manufactured using semiconductor and/or other suitable manufacturing techniques.

In yet another embodiment the catheter comprises a pressure wire (or a guide wire). Examples of commercially available guide wire products that include suitable pressure monitoring elements include, without limitation, the Prime Wire PRESTIGE® pressure guide wire, the Prime Wire® pressure guide wire, and the ComboWire® XT pressure and flow guide wire, each available from Volcano Corporation, as well as the Pressure Wire™ Certus guide wire and the Pressure Wire™ Aeris guide wire, each available from St. Jude Medical, Inc or COMET™ FFR pressure guidewire from Boston Scientific. The pressure wire is also configured to obtain diagnostic information about the coronary vessel. In some instances, the pressure wire is configured to obtain the same diagnostic information as the catheter. In other instances, the pressure wire is configured to obtain different diagnostic information than the catheter, which may include additional diagnostic information, less diagnostic information, and/or alternative diagnostic information. The diagnostic information obtained by the pressure wire includes one or more of pressure, flow (velocity), images (including images obtained using ultrasound (e.g. IVUS), OCT, thermal, and/or other imaging techniques), temperature, and/or combinations thereof.

Similar to the catheter the pressure wire also includes at least one element configured to monitor pressure within the vessel. The pressure monitoring element can take the form a piezo-resistive pressure sensor, a piezo-electric pressure sensor, a capacitive pressure sensor, an electromagnetic pressure sensor, an optical pressure sensor, and/or combinations thereof. In some instances, one or more features of the pressure monitoring element are implemented as a solid-state component manufactured using semiconductor and/or other suitable manufacturing techniques. In a particular embodiment the pressure wire can comprise multiple pressure sensors, e.g. at least 10, at least 20, at least 30, at least 40, at least 50, or more pressure sensors. It is clear that according to such embodiments of the pressure wire, the multiple pressure sensors are provided at different positions along the length of the pressure wire, and thus configured to, even when stationary, after being introduced into the coronary vessel up to the distal end of the coronary vessel, determine a plurality of pressure measurements at different positions along the length of the coronary vessel, or in other words at different positions between the ostium and the distal end of the coronary vessel.

In a particular embodiment the pressure wire is configured to monitor pressure within the vessel while being moved through the lumen of the vessel. In some instances, the pressure wire is configured to be moved through the lumen of the vessel and across the stenosis present in the vessel. In that regard, the pressure wire is positioned distal of the stenosis and moved proximally (i.e. pulled back) across the stenosis to a position proximal of the stenosis in some instances. Movement of the pressure wire can be controlled manually by medical personnel (e.g. hand of a surgeon) in some embodiments. In other preferred embodiments, movement of the pressure wire is controlled automatically by a movement control device (e.g. a pullback device, such as the Trak Back® II or Volcano R-100 Device available from Volcano Corporation). In that regard, the movement control device controls the movement of the pressure wire at a selectable and known speed (e.g. 5.0mm/s, 2.0 mm/s, 1.0 mm/s, 0.5 mm/s, etc.) in some instances. Movement of the pressure wire through the vessel is continuous for each pullback, in some instances. In other instances, the pressure wire is moved stepwise through the vessel (i.e. repeatedly moved a fixed amount of distance and/or a fixed amount of time).

In yet another embodiment the invention provides a system for evaluating coronary artery disease in a patient under hyperaemic conditions, comprising

i) a coronary catheter comprising a pressure sensor, said catheter further comprising a pressure wire comprising at least one pressure sensor,

ii) a computing device in communication with the catheter and the pressure wire, the computing device configured to generate an FFR curve based on a multiple of FFR values (the latter are relative pressure measurements from pressures obtained over the total length of the coronary vessel relative to the pressure in the ostium),

iii) said computer device comprising a computer algorithm which calculates a functional outcome index (FOI) based on the FFR curve, and the correlation of the FFR values obtained in step ii) over the length of the vessel, the computer output displays an FOI value which informs an interventional cardiologist of a treatment option based on the likelihood for the presence of focal or diffuse coronary artery disease in the coronary artery.

In yet another embodiment the invention provides a system for evaluating coronary artery disease in a patient under hyperaemic conditions, comprising

i) a coronary catheter comprising a pressure sensor, said catheter further comprising a pressure wire comprising at least one pressure sensor, said pressure wire is coupled to a motorized device with a fixed pullback speed,

ii) a computing device in communication with the catheter and the pressure wire, the computing device configured to generate an FFR curve based on relative pressure measurements from the pressures obtained in the coronary vessel relative to the pressure in the ostium, and said computing device also co-registers the relative pressure measurements with the positions in the coronary vessel,

iii) said computer device comprising a computer algorithm which calculates a functional outcome index (FOI) based on the FFR curve, and the computer output displays an FOI value which informs a cardiologist of a treatment option based on the likelihood for the presence of focal or diffuse coronary artery disease in the coronary artery.

In the present invention a "system" is equivalent to a "device" or an "apparatus".

A computing device is generally representative of any device suitable for performing the processing and analysis techniques discussed within the present disclosure. In some embodiments, the computing device includes a processor, random access memory, and a storage medium. In that regard, in some particular instances the computing device is programmed to execute steps associated with the data acquisition and analysis described herein. Accordingly, it is understood that any steps related to data acquisition, data processing, calculation of the FOI, instrument control, and/or other processing or control aspects of the present disclosure may be implemented by the computing device using corresponding instructions stored on or in a non-transitory computer readable medium accessible by the computing device. In some instances, the computing device is a console device. In some instances, the computing device is portable (e.g. handheld, on a rolling cart, etc.). Further, it is understood that in some instances the computing device comprises a plurality of computing devices. In that regard, it is particularly understood that the different processing and/or control aspects of the present disclosure may be implemented separately or within predefined groupings using a plurality of computing devices. Any divisions and/or combinations of the processing and/or control aspects described herein across multiple computing devices are within the scope of the present disclosure.

It is understood that any communication pathway between the catheter and the computing device may be utilized, including physical connections (including electrical, optical, and/or fluid connections), wireless connections, and/or combinations thereof. In that regard, it is understood that the connection is wireless in some instances. In some instances, the connection a communication link over a network (e.g. intranet, internet, telecommunications network, and/or other network). In that regard, it is understood that the computing device is positioned remote from an operating area where the catheter is being used in some instances. Having the connection include a connection over a network can facilitate communication between the catheter and the remote computing device regardless of whether the computing device is in an adjacent room, an adjacent building, or in a different state/country. Further, it is understood that the communication pathway between the catheter and the computing device is a secure connection in some instances. Further still, it is understood that, in some instances, the data communicated over one or more portions of the communication pathway between the catheter and the computing device is encrypted.

It is also understood that the FOI value obtained regarding characteristics of the coronary artery disease (predicted to be diffuse, intermediate or focal lesion) as indicated by the FOI value can be compared with or considered in addition to other representations of the lesion or stenosis and/or the vessel (e.g. IVUS (including virtual histology), OCT, ICE, Thermal, Infrared, flow, Doppler flow, and/or other vessel data-gathering modalities) to provide a more complete and/or accurate understanding of the vessel characteristics. For example, in some instances the information regarding characteristics of the lesion or stenosis and/or the vessel as indicated by the FOI value are utilized to confirm information calculated or determined using one or more other vessel data-gathering modalities.

The following examples are provided to better illustrate particular embodiments, and they should not be considered limiting the application. The application is limited only by the claims.

Examples

1.Patient population

From November 2017 to January 2019, 111 patients with 158 vessels were included in two European centers. In 100 vessels (79 patients) motorized FFR pullback analysis was feasible (Figure 1). The mean age was $66\pm10$, 11% were females and 29% diabetics. Target vessels were the left anterior descending artery in 66%, left circumflex in 16% and right coronary artery in 18%. Clinical, angiographic and functional characteristics are shown in Table 1. All patients underwent motorized FFR pullback evaluation. The mean pullback length was $97.9\pm19.6$ mm and the mean duration of adenosine infusion was $3.6\pm0.3$ min. There were no adverse intraprocedural events associated with the motorized FFR pullback. Overall, 984.813 FFR values were used to generate the FFR pullback curves. The mean FFR value derived from the pullbacks was $0.89\pm0.09$ and mean distal FFR was $0.83\pm0.09$. The distribution of FFR values is presented in Figure 2. In 37 vessels (37%), the most distal FFR was $\leq0.80$, 22 patients underwent PCI, 3 CABG and 12 were managed with optimal medical therapy.

2.Visual assessment of the CAD pattern

Anatomically and functional CAD were observed in 85 vessels. In 15 cases, pullback curves were assessed as having no physiological disease despite the presence of anatomical stenosis and were excluded from this analysis. Using coronary angiography alone, 63% of the vessel were classified as having focal CAD, 26% as diffuse disease and 11% as a combination of focal and diffuse CAD. The inter-observer agreement on the pattern of CAD based on conventional angiography alone was moderate (Fleiss' Kappa coefficient 0.45; 95% CI 0.29 to 0.61). After the evaluation of the FFR pullback curve, 53% of the vessels were identified as focal disease, 20% as diffuse disease and 27% showed a combined pattern of pressure drop. The inter-observer agreement based on the physiological CAD pattern was substantial (Fleiss' Kappa 0.76; CI 0.67 to 0.87). Of the patients identified with anatomical focal disease, 26% was reclassified to a diffuse or combined CAD pattern whereas 13% of anatomical diffuse disease was reclassified as focal CAD (Figure 3).

3.Quantitative assessment of CAD pattern

The mean $FFR_{lesion}$ $61.7\pm25\%$ whereas the mean percent vessel length with physiological disease was $59.8\pm21\%$. The %$FFR_{lesion}$ and length with physiological disease stratified by the physiological CAD pattern is shown in Table 2. The correlation between delta FFR pressure drop and percent diameter stenosis was weak (r=0.21, p=0.028; Figure 4). The mean FOI was $0.61\pm0.17$. The mean FOI stratified according to tertiles was $0.43\pm0.09$, $0.61\pm0.04$ and $0.78\pm0.08$. Examples of physiological disease patterns with the computed FOI are shown in Figure 5 and the distribution of FOI with the corresponding %$FFR_{lesion}$ and extent of functional disease is shown in Figure 6.

4.Serial lesions

A total of 25 vessels with anatomically defined serial lesions were present in this cohort. By visually assessment of the FFR pullback curve, 40% of vessels with serial lesions were adjudicated as two focal drops, 52% as a combination of focal and diffuse drop and 8% as diffuse CAD. When the contribution of the serial lesions was combined the %$FFR_{lesion}$ was $70.2\pm20\%$. The %$FFR_{lesion}$ in the proximal lesion was $35.0\pm20\%$ and $34.9\pm19\%$ for the distal lesion (p=0.99). Percent vessel length without physiological disease was $46\pm17\%$. The mean FOI was $0.58\pm0.15$ (range 0.30 to 0.95). A sensitivity analysis including only vessel with distal FFR < 0.80 revealed a similar distribution of the physiological patterns of coronary artery disease and FOI.

## 5. Discussion

### 5.1 Summary of Findings

The main findings to come to the present invention can be summarized as: 1) coronary angiography was inaccurate to assess the pattern and distribution of CAD; 2) using motorized FFR pullbacks 34% of the vessel disease patterns were reclassified (i.e. focal, diffuse or combined) as compared to conventional angiography; 3) the inclusion of the functional component increased the interobserver agreement concerning the identification of the disease pattern; 4) a new computer algorithm was developed to calculate the FOI. The FOI is based on the functional impact of anatomical lesions and the extent of physiological disease discriminated focal and diffuse CAD using a quantitative metric.

The present invention provides a characterization of the physiological patterns of CAD by assessing the distribution of epicardial coronary resistance under hyperaemic conditions in patients with stable coronary artery disease. Using motorized FFR pullbacks, novel insights into the mechanisms of pressure losses in patients with stable CAD are described. Moreover, the co-registration with coronary angiography allowed us to assess the relationship between anatomical and functional findings at the lesion level confirming a moderate correlation between diameter stenosis and pressure gradient. Three physiological CAD patterns were observed, namely, focal, diffuse or a combination of both mechanisms.

### 5.2 Coronary artery disease patterns

The discrepancy between anatomical and physiological significance of coronary disease has been widely recognized. [11] moreover, there is no consensus regarding the definition of diffuse CAD. Several authors have proposed different descriptions of diffuse CAD based extent of atherosclerosis, vessel diameter, number of lesions and appearance of distal run-off. [9, 16, 17] The present analysis extends our knowledge to the contribution epicardial lesions to overall pressure gradients. In this study, 62% of the vessel FFR drop was related to angiographically visible stenosis; in other words, almost 40% of the FFR drop was not related to angiographic narrowing. Moreover, physiological disease was observed 60% of the vessel length whereas percent lesion length 25% of the vessel length. This analysis reassembles the intravascular ultrasound observations of diffuse coronary atherosclerosis with a physiological repercussion in terms of pressure losses along the coronary vessels. Moreover, these findings could be extrapolated to recent randomized clinical trials in the field of coronary physiology. In the present study, the mean distal FFR was $0.83 \pm 0.09$ that is comparable with the ones observed in Define Flair ($0.83 \pm 0.09$) and SWEDEHEART ($0.82 \pm 0.10$). [18, 19] One fourth of the vessels assessed as focal CAD with conventional angiography showed also diffuse physiological disease whereas one out of ten vessels with an anatomical diffuse disease was reclassified as focal CAD using a motorized FFR pullback. The evaluation of the FFR pullback curve reclassified 34% of the vessel CAD patterns. Moreover, the use of coronary physiology increased the inter-observer reproducibility concerning the CAD pattern. Nonetheless, it should be recognized that using a visual evaluation of the FFR pullback curve a discrepant assessment on the CAD pattern was observed in 19% of the vessel.

### 5.3 Implications for Revascularization Strategies

The distribution of coronary atherosclerosis (e.g. focal and diffuse) has been shown to influence clinical-decision making about the revascularization strategy. Patients with anatomically diffuse CAD are often managed conservatively with optimal medical therapy or referred to coronary artery bypass grafting. [20] Interestingly, diffuse disease has been shown to carry an adverse prognosis even in patients undergoing surgery. Diffuse physiological disease in the LAD has been associated with higher rate of left internal mammary artery graft occlusion as compared with focal disease. [17] Moreover, despite the clinical benefit observed PCI in patients with distal vessel FFR <0.80, one third of the patients undergoing PCI remain with a suboptimal FFR post-PCI which is associated with major adverse cardiac events. [21] [3] Focal percutaneous based therapies are likely able to restore coronary physiology and relieve ischemia in cases of focal physiological CAD. However, the clinical benefit of PCI in cases of diffuse CAD can be questioned. [21] [22] In patients managed medically, the assessment of lesion-related gradients might also aid in lesion-based risk stratification; high delta lesions FFR gradients (i.e. > 0.06) has been identified as a hemodynamic predictor of plaque rupture and acute coronary syndromes. [23] On top of contemporary risk stratification using clinical characteristics, luminal and atherosclerotic plaque components, and the presence of ischemia, determining FFR lesion gradients and the physiological pattern of CAD may further refine lesion-based risk stratification. Furthermore, an individualized approach based on the physiological disease at the vessel and lesion level has the potential to improve clinical decision-making and outcomes.

In the present invention, a new physiological metric to objectivize the pattern of CAD was developed. The FOI epitomise the physiological pattern of CAD as focal, diffuse or combined. Rather than trichotomizing the data to define the pattern of CAD, the FOI should be interpreted as a continuous metric. The higher the FOI the more focal CAD and higher the potential gain in epicardial conductance with PCI. The availability of a quantitative metric to characterize CAD patterns under hyperaemic conditions has enabled us to design a clinical trial to investigate the effectiveness of PCI versus optimal medical therapy stratified by the physiological pattern of CAD. This will further personalize treatment strategies in patient with CAD based on coronary physiology.

## 5.4 Serial lesions

Some authors have defined the presence of serial lesions as diffuse CAD. In the current cohort, serial lesions were found in 29% of the vessels. Visually the FFR pullback curve depicted two focal drops in Visually the FFR pullback curve depicted two focal drops in 40%, one focal combined with a diffuse drop in 52% and diffuse disease (no focal FFR drops) in 8%. The FOI ranged from 0.30 to 0.95 depicting the variable physiological repercussion of serial lesions. Physiological interdependency in the coronary tree, the so-called lesion cross-talked, has been described under hyperaemic conditions. [24] We observed that the functional contribution of each lesion in terms of percent delta FFR was similar for proximal and distal lesions. No differences were found concerning percent diameter stenosis or %FFR$_{lesion}$ between the proximal and distal lesion. This finding is likely consequence of the intermediate angiographic disease (mean percent diameter stenosis 45.9 $\pm$ 14.2%) observed in this population which might be insufficient to reduce coronary flow and ameliorate pressure gradients in the distal lesion. [25] [26] [27] In cases of serial lesions, the true FFR gradient can be unmasked by the removal of one lesion and reassessment of FFR. Kim et al. have found that treating the lesion with the greatest delta FFR and reassessing the functional component of the vessel to determine whether further treatment is required is a safe strategy. [28] Also, traditional statistics and machine learning methods have been developed to predict functional outcomes in term of FFR in serial lesions.

## 5.5 Clinical Implications

The adoption of coronary physiology in clinical practice continues to increase after evidence of clinical benefit compared to anatomical guidance and medical therapy, and the development of non-hyperaemic pressure ratios. [29] As the field move forward, refinements in invasive techniques have the potential further improve clinical decision-making and patient selection for revascularization. The characterization of the pattern of coronary artery disease is a necessary step in this direction aiming at predicting which patient benefit the most from PCI, CABG or medical therapy based on the distribution of epicardial resistance. Prediction of functional outcomes after PCI is an important topic and a matter of intense research with non-invasive and invasive methods. [30] Angiography-derived FFR and FFR derived from CT angiography have an inherent advantage given the possibility to provide an FFR value at any point of the coronary tree and therefore characterizing the CAD pattern. [31] [32] It is thus clear that according to such an embodiment, the FFR values at different positions along the length of a coronary vessel could be generated by and/or CT Angiography-derived FFR values acquired from a device configured to provide Angiography-derived FFR values or along the length of the coronary vessel, and/or at any desired point of the coronary tree. These tools will have to demonstrate clinical benefit to be adopted in clinical practice as integral part of the physiological assessment of CAD, refining selection and ultimately improving clinical outcomes of patients with stable coronary artery disease.

## 5.6 Conclusion

Coronary angiography was inaccurate to assess the patterns of CAD. The inclusion of the functional component reclassified 34% of the vessel disease patterns (i.e. focal, diffuse or combined). A new metric, the FOI, based on the functional impact of anatomical lesions and the extent of physiological disease discriminated focal from diffuse CAD has been developed.

## Materials and methods

## 1. Study design

Prospective, multicentre study of patients undergoing clinically-indicated coronary angiography. Fractional flow reserve evaluation was recommended in patients with intermediate coronary lesions defined as visual diameter stenosis between 30% and 70%. A motorized FFR pullback was performed at in all patients. Patients presenting with acute coronary syndromes, previous coronary artery bypass grafting, significant valvular disease, severe obstructive pulmonary disease or asthma bronchial, coronary ostial lesions, with severe tortuosity or severe calcification were excluded. The study was approved by the investigational review board or ethics committee at each participating center.

### 2.Motorized FFR procedure

**FFR** measurements were performed following the recommendations of the Standardization of Fractional Flow Reserve Measurements document. [15] Pressure wire was positioned at least 20 mm distal to the most distal coronary stenosis in vessels more than 2 mm of diameter by visual estimation. Pressure-wire position was recorded using contrast injection. The RadiAnalyzer Xpress (St Jude Medical, Mineapolis, USA) and QUANTIEN Integrated **FFR** System (Abbott Vascular, Illinois, USA) were used to measure invasive coronary pressures. Following intra-coronary nitrates administration, a continuous intra-venous adenosine infusion was given at a dose of 140 $\mu$g/kg/h via a peripheral or central vein to obtain a steady-state hyperaemia for at least 2 minutes. A pullback device (Volcano R 100, San Diego CA, USA), adapted to grip the coronary pressure wire (PressureWire X, St Jude Medical, Mineapolis, USA), was set at a speed of 1 mm/sec to pullback the pressure-wire until the tip of the guiding catheter during continued pressure recording. The maximal pullback length was 13 cm per vessel. If FFR drift (>0.03) was observed, the FFR measurement was repeated.

### 3.Pressure tracing analysis

An FFR value was extracted from the pressure tracing every 10 microns. FFR was defined as the ratio of the moving average of the proximal and distal coronary pressures. Pressure tracings were examined to evaluate quality, curve artefacts and hyperemia stability (Supplementary appendix Figure 1). Absence of functional CAD was defined as distal vessel FFR > 0.95. The pattern of CAD was adjudicated by visual inspection of the FFR pullback curves as focal, diffuse or as a combination of both mechanisms. Also, a quantitative classification of the physiological pattern of CAD was performed based on (1) the functional contribution of the epicardial lesion with respect to the total vessel FFR ($\Delta$lesion FFR / $\Delta$vessel FFR) and (2) the length (mm) of epicardial coronary segments with FFR drops with respect to the total vessel length. The combination of these two ratios, namely, lesion-related pressure drops (%FFR$_{lesion}$) and the extent of functional disease resulted in the functional outcomes index (FOI), a metric that depicts the pattern of CAD (i.e. focality or diffuseness) based on coronary physiology.

$$FOI = \frac{\frac{\Delta\,FFR\;lesion}{\Delta\,FFR\;vessel} + \left(1 - \left(\frac{Length\;with\;FFR\;drop}{Total\;vessel\;length}\right)\right)}{2}$$

Where $\Delta$FFR$_{lesion}$ is defined as the difference between FFR values at the proximal and distal lesion edge of the lesion; $\Delta$FFR$_{vessel}$ as the difference between FFR values between the ostium of the vessel and the most distal FFR measurement, and length with FFR drop defined as the sum of contiguous millimeters with FFR drop $\geq$ 0.0015. The FOI is a continuous metric, values approaching 1.0 represent focal physiological coronary artery disease and value close to 0 diffuse coronary artery disease. In cases with serial lesions, the physiological contribution of each lesion was added to calculate $\Delta$FFR$_{lesion}$. The calculation was performed using an automated and a proprietary algorithm based on the motorized FFR curve.

It is clear that other suitable values for the threshold could be possible for determining the length with FFR drop then the specific value of 0.0015, in which for example the length with FFR drop is defined as the sum of contiguous millimeters with FFR drop $\geq$ said suitable threshold. Or in other words, the length of the functional disease, corresponds to the sum of the length of segments of the coronary vessel with relative pressure drops that are larger than or equal to such a predetermined threshold, of for example a relative pressure drop of 0.0015 per mm of length of the coronary vessel, or any other suitable threshold value.

### 4.Angiographic evaluation

Coronary angiographies were centrally collected and analyzed by an independent core laboratory. The anatomical pattern of coronary artery disease was adjudicated by visual inspection of the target vessel as focal, diffuse or as a combination of both mechanisms. Serial lesions were defined as the presence of two or more narrowings with visual diameter stenosis greater than 50% separated at least by three times the reference vessel diameter. [16] Lesion length was detected by an automated quantitative coronary angiography (QCA) software. Vessel length was defined from the vessel ostium until the position of the pressure wire sensor. Manual correction QCA tracing was recorded. Quantitative coronary angiography analyses were performed with CAAS Workstation 8.1 (Pie Medical Imaging, Maastricht, The Netherlands). Co-registration of coronary angiographies and FFR pullbacks was performed off-line using anatomical landmarks recorded during imaging acquisition.

5.Statistical analysis

Continuous variables with normal distribution are presented as mean plus/minus standard deviation and non-normally distributed variables as median [interquartile range]. Categorical variables as presented as percentages. Agreement on CAD patterns and between observers was assessed using Fleiss' Kappa. Analysis of variance (ANOVA) was used to compared quantitative variables. Correlation between variables was assess by the Pearson moment coefficient. All analyses were performed in R (R Foundation for Statistical Computing, Vienna, Austria) and graphs created with Data Graph 4.3 software (Visual Data Tool Inc).

Table 1. Baseline clinical, angiographic and physiological.

| Clinical characteristics | | N=79 |
|---|---|---|
| Male, n (%) | | 61 (77.2) |
| Age (yrs), mean±SD | | 67.5 ± 9.0 |
| BMI (kg/m$^2$), mean±SD | | 27.5 ± 4.3 |
| Hypertension, n (%) | | 51 (64.6) |
| Diabetes mellitus, n (%) | | 23 (29.1) |
| Hyperlipidemia, n (%) | | 68 (86.1) |
| Smoking, n (%) | | 8 (10.1) |
| Prior myocardial infarction, n (%) | | 12 (15.2) |
| Left ventricular ejection fraction (%), mean±SD | | 57.9 ± 6.7 |
| Creatinine (mg/dl), mean±SD | | 1.02 ± 0.3 |
| Creatinine clearance (ml/min), mean±SD | | 82.5 ± 28.7 |
| Angiographical characteristics | | |
| Vessel, n | | 100 |
| | LAD, n (%) | 66 (66.0) |
| | LCX, n (%) | 16 (16.0) |
| | RCA, n (%) | 18 (18.0) |
| Serial lesions, n (%) | | 25 (25.0) |
| Quantitative coronary angiography | | |
| Lesion, n | | 111* |
| Diameter stenosis (%), mean ± SD | | 45.9 ± 14.2 |
| Diameter stenosis, n (%) | | |
| | <30%, n (%) | 14 (12.6) |
| | ≥30 % and <50 %, n (%) | 54 (48.6) |
| | >50%, n (%) | 43 (38.7) |
| Minimal lumen area (mm$^2$), mean ± SD | | 1.47 ± 0.50 |
| Reference vessel diameter (mm), mean ± SD | | 2.77 ± 0.62 |
| Lesion length (mm), mean ± SD | | 24.6 ± 11.1 |
| QCA tracing contour correction (%), mean ± SD | | 9.6 ± 7.0 |
| Functional characteristics | | n = 85 |
| Distal FFR, mean ± SD | | 0.81 ± 0.08 |
| Distal FFR ≤ 0.80, n (%) | | 37 (44%) |
| Pullback length (mm), mean ± SD | | 98.9 ± 19.3 |
| FFR gradient in vessel, mean ± SD | | 0.18 ± 0.08 |

(continued)

| Functional characteristics | n = 85 |
|---|---|
| FFR gradient in lesions, mean ± SD | 0.12 ± 0.25 |
| %FFR$_{lesion}$ (%), mean ± SD | 61.7 ± 25.0 |
| Length with FFR drop (mm), mean ± SD | 39.3 ± 21.3 |
| Length with FFR drop (mm), mean ± SD | 59.6 ± 25.6 |
| % vessel length with FFR drop (%), mean ± SD | 40.2 ± 21.1 |
| FOI, mean ± SD | 60.7 ± 16.5 |
| *In vessels with distal fractional flow reserve > 0.95. BMI Body mass index. FFR Fractional flow reserve. FOI Functional outcomes index. LAD Left anterior descending artery. LCX Left Circumflex artery. QCA Quantitative coronary angiography. RCA Right coronary artery. SD standard deviation. | |

Table 2. Anatomical and functional characteristics stratified by coronary artery disease pattern.

| | Low FOI tertile | Intermedate FOI tertile | High FOI tertile | p-value |
|---|---|---|---|---|
| Vessels, n | 28 | 28 | 29 | |
| LAD, n | 27 | 23 | 15 | <0.001 |
| LCX, n | 1 | 2 | 8 | 0.032 |
| RCA, n | 0 | 3 | 6 | 0.036 |
| Serial lesions, n (%) | 10 (35.7) | 9 (32.1) | 6 (20.7) | 0.460 |
| Distal FFR, mean ± SD | 0.79 ± 0.06 | 0.81 ± 0.09 | 0.83 ± 0.08 | 0.160 |
| Pullback length (mm) , mean ± SD | 102.8 ± 17.6 | 98.5 ± 19.5 | 95.6 ± 20.6 | 0.371 |
| Diameter stenosis (%), mean ± SD, mean ± SD | 43.7 ± 12.8 | 44.1 ± 12.2 | 49.5 ± 11.8 | 0.144 |
| Vessel FFR gradient, mean ± SD | 0.21 ± 0.06 | 0.18 ± 0.09 | 0.16 ± 0.08 | 0.108 |
| Lesion FFR gradient, mean ± SD | 0.10 ± 0.06 | 0.12 ± 0.09 | 0.14 ± 0.08 | 0.123 |
| %FFR$_{lesion}$, mean ± SD | 42.2 ± 18.2 | 59.5 ± 20.3 | 82.6 ± 17.9 | <0.001 |
| Length with FFR drop (mm), mean ± SD | 57.7 ± 18.7 | 36.6 ± 18.8 | 24.0 ± 10.0 | <0.001 |
| % Vessel length with FFR drop (%), mean ± SD | 57.3 ± 18.1 | 37.8 ± 20.0 | 26.0 ± 11.2 | <0.001 |
| FOI, mean ± SD | 42.5 ± 9.3 | 60.8 ± 3.8 | 78.3 ± 7.9 | <0.001 |
| Abbreviations as with previous table.<br>Figure captions | | | | |

References

1. Gruntzig AR, Senning A and Siegenthaler WE. Nonoperative dilatation of coronary-artery stenosis: percutaneous transluminal coronary angioplasty. The New England journal of medicine. 1979;301:61-8.

2. Tonino PA, De Bruyne B, Pijls NH, Siebert U, Ikeno F, van' t Veer M, Klauss V, Manoharan G, Engstrom T, Oldroyd KG, Ver Lee PN, MacCarthy PA and Fearon WF. Fractional flow reserve versus angiography for guiding percutaneous coronary intervention. The New England journal of medicine. 2009;360:213-24.

3. Xaplanteris P, Fournier S, Pijls NHJ, Fearon WF, Barbato E, Tonino PAL, Engstrom T, Kaab S, Dambrink JH, Rioufol G, Toth GG, Piroth Z, Witt N, Frobert O, Kala P, Linke A, Jagic N, Mates M, Mavromatis K, Samady H, Irimpen A, Oldroyd K, Campo G, Rothenbuhler M, Juni P and De Bruyne B. Five-Year Outcomes with PCI Guided by Fractional Flow Reserve. The New England journal of medicine. 2018;379:250-259.

4. Pijls NH, De Bruyne B, Peels K, Van Der Voort PH, Bonnier HJ, Bartunek JKJJ and Koolen JJ. Measurement of fractional flow reserve to assess the functional severity of coronary-artery stenoses. The New England journal of medicine. 1996;334:1703-8.

5. Fihn SD, Blankenship JC, Alexander KP, Bittl JA, Byrne JG, Fletcher BJ, Fonarow GC, Lange RA, Levine GN, Maddox TM, Naidu SS, Ohman EM and Smith PK. 2014 ACC/AHA/AATS/PCNA/SCAI/STS focused update of the guideline for the diagnosis and management of patients with stable ischemic heart disease: a report of the American College of Cardiology/American Heart Association Task Force on Practice Guidelines, and the American Association for Thoracic Surgery, Preventive Cardiovascular Nurses Association, Society for Cardiovascular Angiography and Interventions, and Society of Thoracic Surgeons. Circulation. 2014;130:1749-67.

6. Neumann FJ, Sousa-Uva M, Ahlsson A, Alfonso F, Banning AP, Benedetto U, Byrne RA, Collet JP, Falk V, Head SJ, Juni P, Kastrati A, Koller A, Kristensen SD, Niebauer J, Richter DJ, Seferovic PM, Sibbing D, Stefanini GG, Windecker S, Yadav R and Zembala MO. 2018 ESC/EACTS Guidelines on myocardial revascularization. European heart journal. 2019;40:87-165.

7. Gould KL. Pressure-flow characteristics of coronary stenoses in unsedated dogs at rest and during coronary vasodilation. Circulation research. 1978;43:242-53.

8. Modi BN, De Silva K, Rajani R, Curzen N and Perera D. Physiology-Guided Management of Serial Coronary Artery Disease: A Review. JAMA cardiology. 2018;3:432-438.

9. De Bruyne B, Hersbach F, Pijls NH, Bartunek J, Bech JW, Heyndrickx GR, Gould KL and Wijns W. Abnormal epicardial coronary resistance in patients with diffuse atherosclerosis but "Normal" coronary angiography. Circulation. 2001;104:2401-6.

10. Gould KL, Nakagawa Y, Nakagawa K, Sdringola S, Hess MJ, Haynie M, Parker N, Mullani N and Kirkeeide R. Frequency and clinical implications of fluid dynamically significant diffuse coronary artery disease manifest as graded, longitudinal, base-to-apex myocardial perfusion abnormalities by noninvasive positron emission tomography. Circulation. 2000;101:1931-9.

11. Tonino PA, Fearon WF, De Bruyne B, Oldroyd KG, Leesar MA, Ver Lee PN, Maccarthy PA, Van't Veer M and Pijls NH. Angiographic versus functional severity of coronary artery stenoses in the FAME study fractional flow reserve versus angiography in multivessel evaluation. Journal of the American College of Cardiology. 2010;55:2816-21.

12. Butler J, Shapiro M, Reiber J, Sheth T, Ferencik M, Kurtz EG, Nichols J, Pena A, Cury RC, Brady TJ and Hoffmann U. Extent and distribution of coronary artery disease: a comparative study of invasive versus noninvasive angiography with computed angiography. American heart journal. 2007;153:378-84.

13. Mintz GS, Popma JJ, Pichard AD, Kent KM, Satler LF, Chuang YC, DeFalco RA and Leon MB. Limitations of angiography in the assessment of plaque distribution in coronary artery disease: a systematic study of target lesion eccentricity in 1446 lesions. Circulation. 1996;93:924-31.

14. Mintz GS, Painter JA, Pichard AD, Kent KM, Satler LF, Popma JJ, Chuang YC, Bucher TA, Sokolowicz LE and Leon MB. Atherosclerosis in angiographically "normal" coronary artery reference segments: an intravascular ultrasound study with clinical correlations. Journal of the American College of Cardiology. 1995;25:1479-85.

15. Toth GG, Johnson NP, Jeremias A, Pellicano M, Vranckx P, Fearon WF, Barbato E, Kern MJ, Pijls NH and De Bruyne B. Standardization of Fractional Flow Reserve Measurements. Journal of the American College of Cardiology. 2016;68:742-53.

16. Sianos G, Morel MA, Kappetein AP, Morice MC, Colombo A, Dawkins K, van den Brand M, Van Dyck N, Russell ME, Mohr FW and Serruys PW. The SYNTAX Score: an angiographic tool grading the complexity of coronary artery disease. EuroIntervention : journal of EuroPCR in collaboration with the Working Group on Interventional Cardiology of the European Society of Cardiology. 2005;1:219-27.

17. Shiono Y, Kubo T, Honda K, Katayama Y, Aoki H, Satogami K, Kashiyama K, Taruya A, Nishiguchi T, Kuroi A, Orii M, Kameyama T, Yamano T, Yamaguchi T, Matsuo Y, Ino Y, Tanaka A, Hozumi T, Nishimura Y, Okamura Y and Akasaka T. Impact of functional focal versus diffuse coronary artery disease on bypass graft patency. International journal of cardiology. 2016;222:16-21.

18. Gotberg M, Christiansen EH, Gudmundsdottir IJ, Sandhall L, Danielewicz M, Jakobsen L, Olsson SE, Ohagen P, Olsson H, Omerovic E, Calais F, Lindroos P, Maeng M, Todt T, Venetsanos D, James SK, Karegren A, Nilsson M, Carlsson J, Hauer D, Jensen J, Karlsson AC, Panayi G, Erlinge D and Frobert O. Instantaneous Wave-free Ratio versus Fractional Flow Reserve to Guide PCI. The New England journal of medicine. 2017;376:1813-1823.

19. Davies JE, Sen S, Dehbi HM, Al-Lamee R, Petraco R, Nijjer SS, Bhindi R, Lehman SJ, Walters D, Sapontis J, Janssens L, Vrints CJ, Khashaba A, Laine M, Van Belle E, Krackhardt F, Bojara W, Going O, Harle T, Indolfi C, Niccoli G, Ribichini F, Tanaka N, Yokoi H, Takashima H, Kikuta Y, Erglis A, Vinhas H, Canas Silva P, Baptista SB, Alghamdi A, Hellig F, Koo BK, Nam CW, Shin ES, Doh JH, Brugaletta S, Alegria-Barrero E, Meuwissen M, Piek JJ, van Royen N, Sezer M, Di Mario C, Gerber RT, Malik IS, Sharp ASP, Talwar S, Tang K, Samady H, Altman J, Seto AH, Singh J, Jeremias A, Matsuo H, Kharbanda RK, Patel MR, Serruys P and Escaned J. Use of the Instantaneous Wave-free Ratio or Fractional Flow Reserve in PCI. The New England journal of medicine. 2017;376:1824-1834.

20. Dourado LOC, Bittencourt MS, Pereira AC, Poppi NT, Dallan LAO, Krieger JE, Cesar LAM and Gowdak LHW. Coronary Artery Bypass Surgery in Diffuse Advanced Coronary Artery Disease: 1-Year Clinical and Angiographic Results. The Thoracic and cardiovascular surgeon. 2018;66:477-482.

21. Piroth Z, Toth GG, Tonino PAL, Barbato E, Aghlmandi S, Curzen N, Rioufol G, Pijls NHJ, Fearon WF, Juni P and De Bruyne B. Prognostic Value of Fractional Flow Reserve Measured Immediately After Drug-Eluting Stent Implantation. Circulation Cardiovascular interventions. 2017;10.

22. Baranauskas A, Peace A, Kibarskis A, Shannon J, Abraitis V, Bajoras V, Bilkis V, Aidietis A, Laucevicius A and Davidavicius G. FFR result post PCI is suboptimal in long diffuse coronary artery disease. EuroIntervention : journal of EuroPCR in collaboration with the Working Group on Interventional Cardiology of the European Society of Cardiology. 2016;12:1473-1480.

23. Lee JM, Choi G, Koo BK, Hwang D, Park J, Zhang J, Kim KJ, Tong Y, Kim HJ, Grady L, Doh JH, Nam CW, Shin ES, Cho YS, Choi SY, Chun EJ, Choi JH, Norgaard BL, Christiansen EH, Niemen K, Otake H, Penicka M, de Bruyne B, Kubo T, Akasaka T, Narula J, Douglas PS, Taylor CA and Kim HS. Identification of High-Risk Plaques Destined to Cause Acute Coronary Syndrome Using Coronary Computed Tomographic Angiography and Computational Fluid Dynamics. JACC Cardiovascular imaging. 2018.

24. Fearon WF, Yong AS, Lenders G, Toth GG, Dao C, Daniels DV, Pijls NHJ and De Bruyne B. The impact of downstream coronary stenosis on fractional flow reserve assessment of intermediate left main coronary artery disease: human validation. JACC Cardiovascular interventions. 2015;8:398-403.

25. Pijls NH, De Bruyne B, Bech GJ, Liistro F, Heyndrickx GR, Bonnier HJ and Koolen JJ. Coronary pressure measurement to assess the hemodynamic significance of serial stenoses within one coronary artery: validation in humans. Circulation. 2000;102:2371-7.

26. De Bruyne B, Pijls NH, Heyndrickx GR, Hodeige D, Kirkeeide R and Gould KL. Pressure-derived fractional flow reserve to assess serial epicardial stenoses: theoretical basis and animal validation. Circulation. 2000;101:1840-7.

27. Modi BN, Ryan M, Chattersingh A, Eruslanova K, Ellis H, Gaddum N, Lee J, Clapp B, Chowienczyk P and Perera D. Optimal Application of Fractional Flow Reserve to Assess Serial Coronary Artery Disease: A 3D-Printed Experimental Study With Clinical Validation. Journal of the American Heart Association. 2018;7:e010279.

28. Kim HL, Koo BK, Nam CW, Doh JH, Kim JH, Yang HM, Park KW, Lee HY, Kang HJ, Cho YS, Youn TJ, Kim SH, Chae IH, Choi DJ, Kim HS, Oh BH and Park YB. Clinical and physiological outcomes of fractional flow reserve-guided percutaneous coronary intervention in patients with serial stenoses within one coronary artery. JACC Cardiovascular interventions. 2012;5:1013-8.

29. Gotberg M, Cook CM, Sen S, Nijjer S, Escaned J and Davies JE. The Evolving Future of Instantaneous Wave-Free Ratio and Fractional Flow Reserve. Journal of the American College of Cardiology. 2017;70:1379-1402.

30. Gosling RD, Morris PD, Lawford PV, Hose DR and Gunn JP. Personalised Fractional Flow Reserve: Novel Concept to Optimise Myocardial Revascularization. EuroIntervention : journal of EuroPCR in collaboration with the Working Group on Interventional Cardiology of the European Society of Cardiology. 2018.

31. Collet C, Katagiri Y, Miyazaki Y, Asano T, Sonck J, van Geuns RJ, Andreini D, Bittencourt MS, Kitslaar P, Tenekeciouglu E, Tijssen JGP, Piek JJ, de Winter RJ, Cosyns B, Rogers C, Zarins CK, Taylor C, Onuma Y and Serruys PW. Impact of Coronary Remodeling on Fractional Flow Reserve. Circulation. 2018;137:747-749.

32. Collet C, Onuma Y, Sonck J, Asano T, Vandeloo B, Kornowski R, Tu S, Westra J, Holm NR, Xu B, de Winter RJ, Tijssen JG, Miyazaki Y, Katagiri Y, Tenekecioglu E, Modolo R, Chichareon P, Cosyns B, Schoors D, Roosens B, Lochy S, Argacha JF, van Rosendael A, Bax J, Reiber JHC, Escaned J, De Bruyne B, Wijns W and Serruys PW. Diagnostic performance of angiography-derived fractional flow reserve: a systematic review and Bayesian meta-analysis. European heart journal. 2018;39:3314-3321.

**Claims**

1. A computer-implemented method for quantifying the patterns of coronary artery functional disease in a coronary vessel from a patient under hyperaemic conditions, comprising the following steps:

    - acquiring a set of relative pressure values obtained from:

        - pressure values obtained at different positions along the coronary vessel between the ostium and the most distal part of the coronary vessel; relative to
        - the pressure at the ostium of the vessel,

    - mapping said set of relative pressure values along the coronary vessel length, and determining:

        - the contribution of the relative pressure drop of the functional disease with respect to the relative pressure drop over the total length of the coronary vessel; and

- the extent of the functional disease, which corresponds to:

- the length of suspected vessel lesions, with respect to the total length of the coronary vessel;
- the length of suspected vessel lesions with relative pressure drops, with respect to the total length of the coronary vessel; or
- the sum of the length of segments of the coronary vessel with relative pressure drops that are larger than or equal to a predetermined threshold, with respect to the total length of the coronary vessel.

2. The method according to claim 1, wherein the method comprises the further step of:

- calculating a functional outcome index (FOI) based on the combination of:

- said contribution of the pressure drop of the functional disease to the pressure drop over the total length of the coronary vessel; and
- said extent of the functional disease.

3. The method according to claim 1 or 2, wherein:

- said contribution of the pressure drop of the functional disease to the pressure drop over the total length of the coronary vessel corresponds to the ratio of:

- the relative pressure drop between the proximal and distal edge of the functional disease, with respect to
- the relative pressure drop between the ostium and the most distal end of the coronary vessel; and

- the extent of the functional disease, corresponds the ratio of:

- the length of the functional disease, with respect to
- the total length of the coronary vessel.

4. The method according to claim 3, wherein:

- the length of the functional disease, corresponds to:

- the length of suspected vessel lesions;
- the length of suspected vessel lesions with relative pressure drops; or
- the sum of the length of segments of the coronary vessel with relative pressure drops that are larger than or equal to a predetermined threshold.

5. The method according to claim any of the claims 1 to 4, wherein the predetermined threshold is equal to a relative pressure drop of 0.0015 per mm of length of the coronary vessel.

6. The method according to any of the preceding claims, wherein the method comprises the steps of:

- acquiring a fractional flow reserve (FFR) pullback curve based on a multiple of FFR values obtained at different positions of the coronary vessel between the ostium and the most distal part of the coronary vessel,
- mapping said multiple of FFR values along the coronary vessel length, and determining:

- the contribution of said FFR drop of the functional disease with respect to the FFR drop over the total length of the coronary vessel; and
- said extent of the functional disease.

7. The method according to claim 6, when dependent on claim 2, wherein the method comprises said step of:

- calculating said functional outcome index (FOI) on the data from the FFR curve, such that the FOI is an expression of at least one of the following functional patterns of coronary artery disease:

- a focal coronary artery disease;
- a diffuse coronary artery disease.

8. The method according to claim 7, wherein the method comprises said step of:

- calculating said functional outcome index (FOI) on the data from the FFR curve based on formula:

$$FOI = \frac{\dfrac{\Delta\, FFR\ lesion}{\Delta\, FFR\ vessel} + \left(1 - \left(\dfrac{Length\ with\ FFR\ drop}{Total\ vessel\ length}\right)\right)}{2}$$

wherein $\Delta FFR_{lesion}$ is defined as the difference between FFR values at the proximal and distal edge of the functional disease; $\Delta FFR_{vessel}$ as the difference between FFR values between the ostium and the most distal part of the coronary vessel; Length with FFR drop is defined as the sum of contiguous millimeters with FFR drop $\geq$ 0.0015; and the total vessel length is the distance between the ostium and the most distant part of the coronary vessel.

9. The method according to claim 8, wherein, when the value of the FOI:

- is higher than 0.7, this indicates the functional pattern of a focal coronary artery disease; and/or
- is lower than 0.4, this indicates the functional pattern of a diffuse coronary artery disease.

10. The method according to any of the preceding claims, wherein said set of multiple of relative pressure values were obtained:

- by means of a manual or motorized pullback of a pressure wire comprising at least one pressure sensor;
- by means of a pressure wire comprising a multiple of built-in pressure sensors;
- from Angiography-derived FFR values along the length of the coronary vessel; and/or
- from CT Angiography-derived FFR values along the length of the coronary vessel.

11. A computer device for evaluating coronary artery disease in a patient under hyperaemic conditions, said computer device configured to generate an FFR curve based on a multiple of FFR values, which are relative pressure measurements from pressures obtained at different positions along the total length of the coronary vessel between the ostium and the most distal part of the coronary vessel, relative to the pressure at the ostium of the coronary vessel, and wherein said computer device is further configured to map said multiple of FFR values along the coronary vessel length, and to determine:

- the contribution of said FFR drop of the functional disease with respect to the FFR drop over the total length of the coronary vessel; and
- said extent of the functional disease, which corresponds to:

- the length of suspected vessel lesions, with respect to the total length of the coronary vessel;
- the length of suspected vessel lesions with relative pressure drops, with respect to the total length of the coronary vessel; or
- the sum of the length of segments of the coronary vessel with relative pressure drops that are larger than or equal to a predetermined threshold, with respect to the total length of the coronary vessel.

12. The computer device according to claim 11, wherein said computer device comprises a computer algorithm configured to calculate a functional outcome index (FOI) based on the combination of:

- said contribution of the pressure drop of the functional disease to the pressure drop over the total length of the coronary vessel; and
- said extent of the functional disease.

13. The computer device according to any of the claims 11 to 12, wherein said computer device comprises a computer algorithm configured to calculate a functional outcome index (FOI) based on the FFR curve and the correlation of the FFR values over the total length of the vessel, the computer output configured to display an FOI value, such that the FOI value is an expression of at least one of the following functional patterns of coronary artery disease:

- a focal coronary artery disease;

- a diffuse coronary artery disease.

14. The computer device according to any of the claims 11 to 13, wherein said computer device comprises a computer algorithm configured to calculate said functional outcome index (FOI) on the data from the FFR curve based on formula:

$$FOI = \frac{\frac{\Delta\,FFR\,lesion}{\Delta\,FFR\,vessel} + \left(1 - \left(\frac{Length\,with\,FFR\,drop}{Total\,vessel\,length}\right)\right)}{2}$$

wherein $\Delta FFR_{lesion}$ is defined as the difference between FFR values at the proximal and distal edge of the functional disease; $\Delta FFR_{vessel}$ as the difference between FFR values between the ostium and the most distal part of the coronary vessel; Length with FFR drop is defined as the sum of contiguous millimeters with FFR drop $\geq 0.0015$; and the total vessel length is the distance between the ostium and the most distant part of the coronary vessel.

15. The computer device according to any of the claims 11 to 14, wherein said computing device is further configured to co-register the relative pressure measurements with the positions in the coronary vessel.

16. A system for evaluating coronary artery disease in a patient under hyperaemic conditions comprising the computer device according to any of the claims 11 to 15, wherein the system further comprises at least one of the following, in communication with the computer device, and configured to generate the multiple FFR values :

- A catheter and a pressure wire comprising at least one pressure sensor,
- A catheter and a pressure wire is coupled to a motorized device with a fixed pullback speed;
- A catheter and a pressure wire comprising a multiple of built-in pressure sensors;
- A device configured to provide Angiography-derived FFR values along the length of the coronary vessel;
- A device configured to provide CT Angiography-derived FFR values along the length of the coronary vessel.

**Patentansprüche**

1. Computerimplementiertes Verfahren zum Quantifizieren der Muster von funktionellen Koronararterienerkrankungen in einem Koronargefäß von einem Patienten unter hyperämischen Bedingungen, umfassend die folgenden Schritte:

- Erfassen eines Satzes von relativen Druckwerten, die hiervon erhalten werden:
- Druckwerte, die an unterschiedlichen Positionen entlang des Koronargefäßes zwischen dem Ostium und dem distalsten Teil des Koronargefäßes erhalten werden; relativ zu
- dem Druck am Ostium des Gefäßes,
- Kartieren des besagten Satzes von relativen Druckwerten entlang der Koronargefäßlänge und Bestimmen hiervon:

- Anteil des relativen Druckabfalls der funktionellen Erkrankung im Hinblick auf den relativen Druckabfall über die Gesamtlänge des Koronargefäßes; und
- Ausmaß der funktionellen Erkrankung, entsprechend diesem:

- Länge von vermuteten Gefäßläsionen im Hinblick auf die Gesamtlänge des Koronargefäßes;
- Länge von vermuteten Gefäßläsionen mit relativen Druckabfällen im Hinblick auf die Gesamtlänge des Koronargefäßes; oder
- Summe der Länge von Segmenten des Koronargefäßes mit relativen Druckabfällen, die größer als oder gleich einem vorbestimmten Schwellwert sind, im Hinblick auf die Gesamtlänge des Koronargefäßes.

2. Verfahren gemäß Anspruch 1, wobei das Verfahren ferner diesen Schritt umfasst:

- Berechnen eines Funktionsergebnis-Index (Functional Outcome Index, FOI) basierend auf der Kombination hiervon:

- Anteil des Druckabfalls der funktionellen Erkrankung am Druckabfall über die Gesamtlänge des Koronargefäßes; und
- Ausmaß der funktionellen Erkrankung.

3. Verfahren nach Anspruch 1 oder 2, wobei:

- der Anteil des Druckabfalls der funktionellen Erkrankung am Druckabfall über die Gesamtlänge des Koronargefäßes diesem Verhältnis entspricht:

  - relativer Druckabfall zwischen dem proximalen und dem distalen Rand der funktionellen Erkrankung im Hinblick auf
  - den relativen Druckabfall zwischen dem Ostium und dem distalsten Ende des Koronargefäßes; und
  - das Ausmaß der funktionellen Erkrankung dem Verhältnis hiervon entspricht:

    - Länge der funktionellen Erkrankung im Hinblick auf
    - Gesamtlänge des Koronargefäßes.

4. Verfahren nach Anspruch 3, wobei:

  - die Länge der funktionellen Erkrankung diesem entspricht:

    - Länge von vermuteten Gefäßläsionen;
    - Länge von vermuteten Gefäßläsionen mit relativen Druckabfällen; oder
    - Summe der Länge von Segmenten des Koronargefäßes mit relativen Druckabfällen, die größer als oder gleich einem vorbestimmten Schwellwert sind.

5. Verfahren gemäß einem der Ansprüche 1 bis 4, wobei der vorbestimmte Schwellwert gleich einem relativen Druckabfall von 0,0015 pro mm Länge des Koronargefäßes ist.

6. Verfahren gemäß einem der vorstehenden Ansprüche, wobei das Verfahren diese Schritte umfasst:

  - Erfassen einer fraktionalen Flussreserve (Fractional Flow Reserve, FFR)-Rückzugskurve basierend auf mehreren FFR-Werten, die an unterschiedlichen Positionen des Koronargefäßes zwischen dem Ostium und dem distalsten Teil des Koronargefäßes erhalten werden,
  - Kartieren der mehreren FFR-Werte entlang der Koronargefäßlänge und Bestimmen hiervon:

    - Anteil des FFR-Abfalls der funktionellen Erkrankung im Hinblick auf den FFR-Abfall über die Gesamtlänge des Koronargefäßes; und
    - Ausmaß der funktionellen Erkrankung.

7. Verfahren gemäß Anspruch 6 bei Abhängigkeit von Anspruch 2, wobei das Verfahren diesen Schritt umfasst:

  - Berechnen des Funktionsergebnis-Index (Functional Outcome Index, FOI) für die Daten aus der FFR-Kurve, sodass der FOI ein Ausdruck von wenigstens einem der folgenden Funktionsmuster einer Koronararterienerkrankung ist:

    - fokale Koronararterienerkrankung;
    - diffuse Koronararterienerkrankung.

8. Verfahren gemäß Anspruch 7, wobei das Verfahren diesen Schritt umfasst:

  - Berechnen des Funktionsergebnis-Index (Functional Outcome Index, FOI) für die Daten aus der FFR-Kurve basierend auf der Formel:

$$FOI = \frac{\frac{\Delta FFR - L\ddot{a}sion}{\Delta FFR - Gef\ddot{a}\ss} + \left(1 - \left(\frac{L\ddot{a}nge\ mit\ FFR - Abfall}{Gesamtgef\ddot{a}\ssl\ddot{a}nge}\right)\right)}{2}$$

wobei $\Delta FFR_{Läsion}$ definiert ist als die Differenz zwischen FFR-Werten am proximalen und distalen Rand der funktionellen Erkrankung; $\Delta FFR_{Gefäß}$ als die Differenz zwischen FFR-Werten zwischen dem Ostium und dem distalsten Teil des Koronargefäßes; Länge mit FFR-Abfall ist definiert als die Summe von zusammenhängenden Millimetern mit einem FFR-Abfall $\geq 0,0015$; und die Gesamtgefäßlänge ist der Abstand zwischen dem Ostium und dem am weitesten entfernten Teil des Koronargefäßes.

9. Verfahren gemäß Anspruch 8, wobei, wenn der Wert des FOI:

- höher als 0,7 ist, dies das Funktionsmuster einer fokalen Koronararterienerkrankung angibt; und/oder
- niedriger als 0,4 ist, dies das Funktionsmuster einer diffusen Koronararterienerkrankung angibt.

10. Verfahren gemäß einem der vorstehenden Ansprüche, wobei der Satz von mehreren relativen Druckwerten wie folgt erhalten wurde:

- mittels eines manuellen oder motorisierten Rückzugs eines Druckdrahtes, umfassend wenigstens einen Drucksensor;
- mittels eines Druckdrahtes, umfassend mehrere integrierte Drucksensoren;
- von Angiografie-abgeleiteten FFR-Werten entlang der Länge des Koronargefäßes; und/oder
- von CT-Angiografie-abgeleiteten FFR-Werten entlang der Länge des Koronargefäßes.

11. Computervorrichtung zum Auswerten einer Koronararterienerkrankung in einem Patienten unter hyperämischen Bedingungen, wobei die Computervorrichtung dazu ausgelegt ist, eine FFR-Kurve basierend auf mehreren FFR-Werten zu generieren, bei denen es sich um relative Druckmessungen von Drücken handelt, die an unterschiedlichen Positionen entlang der Gesamtlänge des Koronargefäßes zwischen dem Ostium und dem distalsten Teil des Koronargefäßes erhalten werden, relativ zu dem Druck am Ostium des Koronargefäßes, und wobei die Computervorrichtung ferner dazu ausgelegt ist, die mehreren FFR-Werte entlang der Koronargefäßlänge zu kartieren und dies zu bestimmen:

- Anteil des FFR-Abfalls der funktionellen Erkrankung im Hinblick auf den FFR-Abfall über die Gesamtlänge des Koronargefäßes; und
- Ausmaß der funktionellen Erkrankung, was Folgendem entspricht:

- Länge von vermuteten Gefäßläsionen im Hinblick auf die Gesamtlänge des Koronargefäßes;
- Länge von vermuteten Gefäßläsionen mit relativen Druckabfällen im Hinblick auf die Gesamtlänge des Koronargefäßes; oder
- Summe der Länge von Segmenten des Koronargefäßes mit relativen Druckabfällen, die größer als oder gleich einem vorbestimmten Schwellwert sind, im Hinblick auf die Gesamtlänge des Koronargefäßes.

12. Computervorrichtung gemäß Anspruch 11, wobei die Computervorrichtung einen Computeralgorithmus umfasst, der dazu ausgelegt ist, einen Funktionsergebnis-Index (Functional Outcome Index, FOI) basierend auf der Kombination hiervon zu berechnen:

- Anteil des Druckabfalls der funktionellen Erkrankung am Druckabfall über die Gesamtlänge des Koronargefäßes; und
- Ausmaß der funktionellen Erkrankung.

13. Computervorrichtung gemäß einem der Ansprüche 11 bis 12, wobei die Computervorrichtung einen Computeralgorithmus umfasst, der dazu ausgelegt ist, einen Funktionsergebnis-Index (Functional Outcome Index, FOI) basierend auf der FFR-Kurve und der Korrelation der FFR-Werte über die Gesamtlänge des Gefäßes zu berechnen, wobei die Computerausgabe dazu ausgelegt ist, einen FOI-Wert anzuzeigen, sodass der FOI-Wert ein Ausdruck von wenigstens einem der folgenden Funktionsmuster einer Koronararterienerkrankung ist:

- fokale Koronararterienerkrankung;
- diffuse Koronararterienerkrankung.

14. Computervorrichtung gemäß einem der Ansprüche 11 bis 13, wobei die Computervorrichtung einen Computeralgorithmus umfasst, der dazu ausgelegt ist, den Funktionsergebnis-Index (Functional Outcome Index, FOI) für die Daten aus der FFR-Kurve basierend auf dieser Formel zu berechnen:

$$FOI = \frac{\frac{\Delta\,FFR-L\ddot{a}sion}{\Delta\,FFR-Gef\ddot{a}\text{ß}} + \left(1 - \left(\frac{L\ddot{a}nge\ mit\ FFR-Abfall}{Gesamtgef\ddot{a}\text{ß}l\ddot{a}nge}\right)\right)}{2}$$

wobei $\Delta FFR_{L\ddot{a}sion}$ definiert ist als die Differenz zwischen FFR-Werten am proximalen und distalen Rand der funktionellen Erkrankung; $\Delta FFR_{Gef\ddot{a}\text{ß}}$ als die Differenz zwischen FFR-Werten zwischen dem Ostium und dem distalsten Teil des Koronargefäßes; Länge mit FFR-Abfall ist definiert als die Summe von zusammenhängenden Millimetern mit einem FFR-Abfall $\geq 0{,}0015$; und die Gesamtgefäßlänge ist der Abstand zwischen dem Ostium und dem am weitesten entfernten Teil des Koronargefäßes.

15. Computervorrichtung gemäß einem der Ansprüche 11 bis 14, wobei die Computervorrichtung ferner dazu ausgelegt ist, die relativen Druckmessungen zusammen mit den Positionen in dem Koronargefäß zu registrieren.

16. System zum Auswerten einer Koronararterienerkrankung in einem Patienten unter hyperämischen Bedingungen, umfassend die Computervorrichtung gemäß einem der Ansprüche 11 bis 15, wobei das System ferner wenigstens eines der folgenden umfasst, in Kommunikation mit der Computervorrichtung und dazu ausgelegt, die mehreren FFR-Werte zu generieren:

   - Katheter und Druckdraht, umfassend wenigstens einen Drucksensor,
   - Katheter und Druckdraht, gekoppelt an eine motorisierte Vorrichtung mit einer festen Rückzugsgeschwindigkeit;
   - Katheter und Druckdraht, umfassend mehrere integrierte Drucksensoren;
   - Vorrichtung, dazu ausgelegt, Angiografieabgeleitete FFR-Werte entlang der Länge des Koronargefäßes bereitzustellen;
   - Vorrichtung, dazu ausgelegt, CT-Angiografieabgeleitete FFR-Werte entlang der Länge des Koronargefäßes bereitzustellen.


**Revendications**

1. Procédé mis en œuvre par ordinateur permettant de quantifier les schémas de la maladie fonctionnelle des artères coronaires dans un vaisseau coronaire d'un patient dans des conditions d'hyperémie, comprenant les étapes suivantes :

   - l'acquisition d'un ensemble de valeurs de pression relative obtenues à partir de :

      - valeurs de pression obtenues à différentes positions le long du vaisseau coronaire entre l'ostium et la partie la plus distale du vaisseau coronaire ; relative à
      - la pression au niveau de l'ostium du vaisseau,
      - la cartographie dudit ensemble de valeurs de pression relative le long de la longueur du vaisseau coronaire, et la détermination de :

         - la contribution de la chute de pression relative de la maladie fonctionnelle par rapport à la chute de pression relative sur la longueur totale du vaisseau coronaire ; et
         - l'étendue de la maladie fonctionnelle, qui correspond à :

            - la longueur de lésions vasculaires suspectes, par rapport à la longueur totale du vaisseau coronaire ;
            - la longueur de lésions vasculaires suspectes avec des chutes de pression relative, par rapport à la longueur totale du vaisseau coronaire ; ou
            - la somme des longueurs de segments du vaisseau coronaire avec des chutes de pression relative qui sont supérieures ou égales à un seuil prédéterminé, par rapport à la longueur totale du vaisseau coronaire.

2. Procédé selon la revendication 1, dans lequel le procédé comprend l'étape supplémentaire de :

   - calcul d'un indice de résultat fonctionnel (IRF) basé sur la combinaison de :

- ladite contribution de la chute de pression de la maladie fonctionnelle à la chute de pression sur la longueur totale du vaisseau coronaire ; et
- ladite étendue de la maladie fonctionnelle.

3. Procédé selon la revendication 1 ou 2, dans lequel :

- ladite contribution de la chute de pression de la maladie fonctionnelle à la chute de pression sur la longueur totale du vaisseau coronaire correspond au ratio de :

- la chute de pression relative entre le bord proximal et distal de la maladie fonctionnelle, par rapport à
- la chute de pression relative entre l'ostium et l'extrémité la plus distale du vaisseau coronaire ; et
- l'étendue de la maladie fonctionnelle, qui correspond au ratio de :

- la longueur de la maladie fonctionnelle, par rapport à
- la longueur totale du vaisseau coronaire.

4. Procédé selon la revendication 3, dans lequel :

- la longueur de la maladie fonctionnelle correspond à :

- la longueur des lésions vasculaires suspectes ;
- la longueur des lésions vasculaires suspectes avec les chutes de pression relative ; ou
- la somme des longueurs de segments du vaisseau coronaire avec des chutes de pression relative qui sont supérieures ou égales à un seuil prédéterminé.

5. Procédé selon l'une quelconque des revendications 1 à 4, dans lequel le seuil prédéterminé est égal à une chute de pression relative de 0,0015 par mm de longueur du vaisseau coronaire.

6. Procédé selon l'une quelconque des revendications précédentes, dans lequel le procédé comprend les étapes de :

- acquisition d'une courbe de retrait de réserve de débit fractionnaire (FFR) basée sur un multiple de valeurs de FFR obtenues à différentes positions du vaisseau coronaire entre l'ostium et la partie la plus distale du vaisseau coronaire,
- cartographie dudit multiple de valeurs de FFR le long de la longueur du vaisseau coronaire, et la détermination de :

- la contribution de ladite chute de FFR de la maladie fonctionnelle par rapport à la chute de FFR sur la longueur totale du vaisseau coronaire ; et
- ladite étendue de la maladie fonctionnelle.

7. Procédé selon la revendication 6, lorsqu'elle dépend de la revendication 2, dans lequel le procédé comprend ladite étape de :

- calcul dudit indice de résultat fonctionnel (IRF) sur les données de la courbe FFR, de telle sorte que l'IRF soit l'expression d'au moins l'un des schémas fonctionnels suivants de la maladie des artères coronaires :

- une maladie des artères coronaires focale ;
- une maladie des artères coronaires diffuse.

8. Procédé selon la revendication 7, dans lequel le procédé comprend ladite étape de :

- calcul dudit indice de résultat fonctionnel (IRF) sur les données de la courbe FFR sur la base de la formule :

$$IRF = \frac{\dfrac{\Delta\,FFR\;lésion}{\Delta\,FFR\;vaisseau} + \left(1 - \left(\dfrac{Longueur\;avec\;chute\;de\;FFR}{Longueur\;totale\;du\;vaisseau}\right)\right)}{2}$$

dans laquelle ΔFFR$_{lésion}$ est définie comme la différence entre les valeurs de FFR aux bords proximal et distal de la maladie fonctionnelle ; ΔFFR$_{vessel}$ comme la différence entre les valeurs de FFR entre l'ostium et la partie la plus distale du vaisseau coronaire ; Longueur avec chute de FFR est définie comme la somme de millimètres contigus avec une chute de FFR ≥ 0,0015 ; et Longueur totale du vaisseau est la distance entre l'ostium et la partie la plus éloignée du vaisseau coronaire.

9. Procédé selon la revendication 8, dans lequel, lorsque la valeur de l'IRF :

- est supérieure à 0,7, cela indique le schéma fonctionnel d'une maladie des artères coronaires focale ; et/ou
- est inférieure à 0,4, cela indique le schéma fonctionnel d'une maladie des artères coronaires diffuse.

10. Procédé selon l'une quelconque des revendications précédentes, dans lequel ledit ensemble de multiples de valeurs de pression relative a été obtenu :

- au moyen d'un retrait manuel ou motorisé d'un fil de pression comprenant au moins un capteur de pression ;
- au moyen d'un fil de pression comprenant de multiples capteurs de pression intégrés ;
- à partir des valeurs de FFR dérivées d'une angiographie le long de la longueur du vaisseau coronaire ; et/ou
- à partir de valeurs de FFR dérivées d'une angiographie TDM le long de la longueur du vaisseau coronaire.

11. Dispositif informatique permettant d'évaluer la maladie des artères coronaires chez un patient dans des conditions d'hyperémie, ledit dispositif informatique étant configuré pour générer une courbe FFR basée sur un multiple de valeurs de FFR, qui sont des mesures de pression relative provenant de pressions obtenues à différentes positions le long de la longueur totale du vaisseau coronaire entre l'ostium et la partie la plus distale du vaisseau coronaire, relative à la pression à l'ostium du vaisseau coronaire, et dans lequel ledit dispositif informatique est en outre configuré pour cartographier ledit multiple de valeurs de FFR sur la longueur du vaisseau coronaire, et pour déterminer :

- la contribution de ladite chute de FFR de la maladie fonctionnelle par rapport à la chute de FFR sur la longueur totale du vaisseau coronaire ; et
- ladite étendue de la maladie fonctionnelle, qui correspond à :

- la longueur de lésions vasculaires suspectes, par rapport à la longueur totale du vaisseau coronaire ;
- la longueur de lésions vasculaires suspectes avec des chutes de pression relative, par rapport à la longueur totale du vaisseau coronaire ; ou
- la somme des longueurs de segments du vaisseau coronaire avec des chutes de pression relative qui sont supérieures ou égales à un seuil prédéterminé, par rapport à la longueur totale du vaisseau coronaire.

12. Dispositif informatique selon la revendication 11, dans lequel ledit dispositif informatique comprend un algorithme informatique configuré pour calculer un indice de résultat fonctionnel (IRF) basé sur la combinaison de :

- ladite contribution de la chute de pression de la maladie fonctionnelle à la chute de pression sur la longueur totale du vaisseau coronaire ; et
- ladite étendue de la maladie fonctionnelle.

13. Dispositif informatique selon l'une quelconque des revendications 11 à 12, dans lequel ledit dispositif informatique comprend un algorithme informatique configuré pour calculer un indice de résultat fonctionnel (IRF) sur la base de la courbe FFR et de la corrélation des valeurs de FFR sur la longueur totale du vaisseau, la sortie informatique étant configurée pour afficher une valeur d'IRF, de telle sorte que la valeur d'IRF soit une expression d'au moins l'un des schémas fonctionnels suivants de la maladie des artères coronaires :

- une maladie des artères coronaires focale ;
- une maladie des artères coronaires diffuse.

14. Dispositif informatique selon l'une quelconque des revendications 11 à 13, dans lequel ledit dispositif informatique comprend un algorithme informatique configuré pour calculer ledit indice de résultat fonctionnel (IRF) sur les données de la courbe FFR sur la base de la formule :

$$IRF \ = \ \frac{\frac{\Delta \ FFR \ lésion}{\Delta \ FFR \ vaisseau} + \left(1 - \left(\frac{Longueur \ avec \ chute \ de \ FFR}{Longueur \ totale \ du \ vaisseau}\right)\right)}{2}$$

dans laquelle $\Delta FFR_{lésion}$ est définie comme la différence entre les valeurs de FFR aux bords proximal et distal de la maladie fonctionnelle ; $\Delta FFR_{vessel}$ comme la différence entre les valeurs de FFR entre l'ostium et la partie la plus distale du vaisseau coronaire ; Longueur avec chute de FFR est définie comme la somme de millimètres contigus avec une chute de FFR $\geq 0,0015$ ; et Longueur totale du vaisseau est la distance entre l'ostium et la partie la plus éloignée du vaisseau coronaire.

15. Dispositif informatique selon l'une quelconque des revendications 11 à 14, dans lequel ledit dispositif informatique est en outre configuré pour co-enregistrer les mesures de pression relative avec les positions dans le vaisseau coronaire.

16. Système d'évaluation de la maladie des artères coronaires chez un patient dans des conditions d'hyperémie comprenant le dispositif informatique selon l'une quelconque des revendications 11 à 15, dans lequel le système comprend en outre au moins l'un des éléments suivants, en communication avec le dispositif informatique, et configuré pour générer les valeurs de FFR multiples :

- un cathéter et un fil de pression comprenant au moins un capteur de pression,
- un cathéter et un fil de pression couplés à un dispositif motorisé ayant une vitesse de retrait fixe ;
- un cathéter et un fil de pression comprenant de multiples capteurs de pression intégrés ;
- un dispositif configuré pour fournir des valeurs de FFR dérivées d'une angiographie le long de la longueur du vaisseau coronaire ;
- un dispositif configuré pour fournir des valeurs de FFR dérivées d'une angiographie TDM le long de la longueur du vaisseau coronaire.

Means and devices for assessing coronary artery disease

159 vessel (117 patients) undergoing motorized FFR pullback evaluation between Nov 2017 and Jan 2019

Exclusions (59 vessels):
- Inadequate pullback (19).
- File damage (4)
- Unstable adenosine (9).
- Pressure tracing artefacts
  - Wedging (8)
  - Loss of dicrotic notch (3)
  - Artefact (7)
- Others (9)

100 vessels (79 patients) with adequate motorized FFR pullback and pressure tracings for analysis

Pullback tracing without functional disease (15 vessels)*

85 vessels (111 lesions) with 845.147 FFR values Mean FFR derived from pullback 0.88 ± 0.09 Mean distal FFR 0.81 ± 0.08

# FIG. 1

FIG. 2

FFR Fractional flow reserve. IQR Interquartile range.

Patterns of Coronary Artery Disease

FIG. 3

FFR Fractional flow reserve. QCA Quantitative coronary angiography.

# FIG. 4

FOI = Functional Outcome Index.

FIG. 5

34

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20160157787 A
- US 20160008084 A
- US 2011071404 A

### Non-patent literature cited in the description

- **GRUNTZIG AR ; SENNING A ; SIEGENTHALER WE**. Nonoperative dilatation of coronary-artery stenosis: percutaneous transluminal coronary angioplasty. *The New England journal of medicine*, 1979, vol. 301, 61-8
- **TONINO PA ; DE BRUYNE B ; PIJLS NH ; SIEBERT U ; IKENO F ; VAN' T VEER M ; KLAUSS V ; MANOHARAN G ; ENGSTROM T ; OLDROYD KG**. Fractional flow reserve versus angiography for guiding percutaneous coronary intervention. *The New England journal of medicine.*, 2009, vol. 360, 213-24
- **XAPLANTERIS P ; FOURNIER S ; PIJLS NHJ ; FEARON WF ; BARBATO E ; TONINO PAL ; ENGSTROM T ; KAAB S ; DAMBRINK JH ; RIOUFOL G**. Five-Year Outcomes with PCI Guided by Fractional Flow Reserve. *The New England journal of medicine.*, 2018, vol. 379, 250-259
- **PIJLS NH ; DE BRUYNE B ; PEELS K ; VAN DER VOORT PH ; BONNIER HJ ; BARTUNEK JKJJ ; KOOLEN JJ**. Measurement of fractional flow reserve to assess the functional severity of coronary-artery stenoses.. *The New England journal of medicine.*, 1996, vol. 334, 1703-8
- **FIHN SD ; BLANKENSHIP JC ; ALEXANDER KP ; BITTL JA ; BYRNE JG ; FLETCHER BJ ; FONAROW GC ; LANGE RA ; LEVINE GN ; MADDOX TM**. 2014 ACC/AHA/AATS/PCNA/SCAI/STS focused update of the guideline for the diagnosis and management of patients with stable ischemic heart disease: a report of the American College of Cardiology/American Heart Association Task Force on Practice Guidelines, and the American Association for Thoracic Surgery, Preventive Cardiovascular Nurses Association, Society for Cardiovascular Angiography and Interventions, and Society of Thoracic Surgeons.. *Circulation.*, 2014, vol. 130, 1749-67
- **NEUMANN FJ ; SOUSA-UVA M ; AHLSSON A ; ALFONSO F ; BANNING AP ; BENEDETTO U ; BYRNE RA ; COLLET JP ; FALK V ; HEAD SJ**. 2018 ESC/EACTS Guidelines on myocardial revascularization. *European heart journal.*, 2019, vol. 40, 87-165
- **GOULD KL**. Pressure-flow characteristics of coronary stenoses in unsedated dogs at rest and during coronary vasodilation. *Circulation research.*, 1978, vol. 43, 242-53
- **MODI BN ; DE SILVA K ; RAJANI R ; CURZEN N ; PERERA D**. Physiology-Guided Management of Serial Coronary Artery Disease: A Review.. *JAMA cardiology.*, 2018, vol. 3, 432-438
- **DE BRUYNE B ; HERSBACH F ; PIJLS NH ; BARTUNEK J ; BECH JW ; HEYNDRICKX GR ; GOULD KL ; WIJNS W**. Abnormal epicardial coronary resistance in patients with diffuse atherosclerosis but "Normal" coronary angiography. *Circulation*, 2001, vol. 104, 2401-6
- **GOULD KL ; NAKAGAWA Y ; NAKAGAWA K ; SDRINGOLA S ; HESS MJ ; HAYNIE M ; PARKER N ; MULLANI N ; KIRKEEIDE R**. Frequency and clinical implications of fluid dynamically significant diffuse coronary artery disease manifest as graded, longitudinal, base-to-apex myocardial perfusion abnormalities by noninvasive positron emission tomography. *Circulation*, 2000, vol. 101, 1931-9
- **TONINO PA ; FEARON WF ; DE BRUYNE B ; OLDROYD KG ; LEESAR MA ; VER LEE PN ; MACCARTHY PA ; VAN'T VEER M ; PIJLS NH**. Angiographic versus functional severity of coronary artery stenoses in the FAME study fractional flow reserve versus angiography in multivessel evaluation. *Journal of the American College of Cardiology.*, 2010, vol. 55, 2816-21
- **BUTLER J ; SHAPIRO M ; REIBER J ; SHETH T ; FERENCIK M ; KURTZ EG ; NICHOLS J ; PENA A ; CURY RC ; BRADY TJ**. Extent and distribution of coronary artery disease: a comparative study of invasive versus noninvasive angiography with computed angiography. *American heart journal.*, 2007, vol. 153, 378-84
- **MINTZ GS ; POPMA JJ ; PICHARD AD ; KENT KM ; SATLER LF ; CHUANG YC ; DEFALCO RA ; LEON MB**. Limitations of angiography in the assessment of plaque distribution in coronary artery disease: a systematic study of target lesion eccentricity in 1446 lesions. *Circulation*, 1996, vol. 93, 924-31

- **MINTZ GS ; PAINTER JA ; PICHARD AD ; KENT KM ; SATLER LF ; POPMA JJ ; CHUANG YC ; BUCHER TA ; SOKOLOWICZ LE ; LEON MB**. Atherosclerosis in angiographically ''normal'' coronary artery reference segments: an intravascular ultrasound study with clinical correlations. *Journal of the American College of Cardiology.*, 1995, vol. 25, 1479-85
- **TOTH GG ; JOHNSON NP ; JEREMIAS A ; PELLICANO M ; VRANCKX P ; FEARON WF ; BARBATO E ; KERN MJ ; PIJLS NH ; DE BRUYNE B**. Standardization of Fractional Flow Reserve Measurements. *Journal of the American College of Cardiology.*, 2016, vol. 68, 742-53
- **SIANOS G ; MOREL MA ; KAPPETEIN AP ; MORICE MC ; COLOMBO A ; DAWKINS K ; VAN DEN BRAND M ; VAN DYCK N ; RUSSELL ME ; MOHR FW**. The SYNTAX Score: an angiographic tool grading the complexity of coronary artery disease. *EuroIntervention : journal of EuroPCR in collaboration with the Working Group on Interventional Cardiology of the European Society of Cardiology*, 2005, vol. 1, 219-27
- **SHIONO Y ; KUBO T ; HONDA K ; KATAYAMA Y ; AOKI H ; SATOGAMI K ; KASHIYAMA K ; TARUYA A ; NISHIGUCHI T ; KUROI A**. Impact of functional focal versus diffuse coronary artery disease on bypass graft patency. *International journal of cardiology.*, 2016, vol. 222, 16-21
- **GOTBERG M ; CHRISTIANSEN EH ; GUDMUNDSDOTTIR IJ ; SANDHALL L ; DANIELEWICZ M ; JAKOBSEN L ; OLSSON SE ; OHAGEN P ; OLSSON H ; OMEROVIC E**. Instantaneous Wave-free Ratio versus Fractional Flow Reserve to Guide PCI. *The New England journal of medicine.*, 2017, vol. 376, 1813-1823
- **DAVIES JE ; SEN S ; DEHBI HM ; AL-LAMEE R ; PETRACO R ; NIJJER SS ; BHINDI R ; LEHMAN SJ ; WALTERS D ; SAPONTIS J**. Use of the Instantaneous Wave-free Ratio or Fractional Flow Reserve in PCI. *The New England journal of medicine*, 2017, vol. 376, 1824-1834
- **DOURADO LOC ; BITTENCOURT MS ; PEREIRA AC ; POPPI NT ; DALLAN LAO ; KRIEGER JE ; CESAR LAM ; GOWDAK LHW**. Coronary Artery Bypass Surgery in Diffuse Advanced Coronary Artery Disease: 1-Year Clinical and Angiographic Results. *The Thoracic and cardiovascular surgeon.*, 2018, vol. 66, 477-482
- **PIROTH Z ; TOTH GG ; TONINO PAL ; BARBATO E ; AGHLMANDI S ; CURZEN N ; RIOUFOL G ; PIJLS NHJ ; FEARON WF ; JUNI P**. Prognostic Value of Fractional Flow Reserve Measured Immediately After Drug-Eluting Stent Implantation. *Circulation Cardiovascular interventions*, 2017, vol. 10
- **BARANAUSKAS A ; PEACE A ; KIBARSKIS A ; SHANNON J ; ABRAITIS V ; BAJORAS V ; BILKIS V ; AIDIETIS A ; LAUCEVICIUS A ; DAVIDAVICIUS G**. FFR result post PCI is suboptimal in long diffuse coronary artery disease. *EuroIntervention : journal of EuroPCR in collaboration with the Working Group on Interventional Cardiology of the European Society of Cardiology.*, 2016, vol. 12, 1473-1480
- **LEE JM ; CHOI G ; KOO BK ; HWANG D ; PARK J ; ZHANG J ; KIM KJ ; TONG Y ; KIM HJ ; GRADY L**. Identification of High-Risk Plaques Destined to Cause Acute Coronary Syndrome Using Coronary Computed Tomographic Angiography and Computational Fluid Dynamics. *JACC Cardiovascular imaging*, 2018
- **FEARON WF ; YONG AS ; LENDERS G ; TOTH GG ; DAO C ; DANIELS DV ; PIJLS NHJ ; DE BRUYNE B.** The impact of downstream coronary stenosis on fractional flow reserve assessment of intermediate left main coronary artery disease: human validation.. *JACC Cardiovascular interventions.*, 2015, vol. 8, 398-403
- **PIJLS NH ; DE BRUYNE B ; BECH GJ ; LIISTRO F ; HEYNDRICKX GR ; BONNIER HJ ; KOOLEN JJ.** Coronary pressure measurement to assess the hemodynamic significance of serial stenoses within one coronary artery: validation in humans. *Circulation.*, 2000, vol. 102, 2371-7
- **DE BRUYNE B ; PIJLS NH ; HEYNDRICKX GR ; HODEIGE D ; KIRKEEIDE R ; GOULD KL**. Pressure-derived fractional flow reserve to assess serial epicardial stenoses: theoretical basis and animal validation. *Circulation*, 2000, vol. 101, 1840-7
- **MODI BN ; RYAN M ; CHATTERSINGH A ; ERUSLANOVA K ; ELLIS H ; GADDUM N ; LEE J ; CLAPP B ; CHOWIENCZYK P ; PERERA D**. Optimal Application of Fractional Flow Reserve to Assess Serial Coronary Artery Disease: A 3D-Printed Experimental Study With Clinical Validation. *Journal of the American Heart Association*, 2018, vol. 7, e010279
- **KIM HL ; KOO BK ; NAM CW ; DOH JH ; KIM JH ; YANG HM ; PARK KW ; LEE HY ; KANG HJ ; CHO YS**. Clinical and physiological outcomes of fractional flow reserve-guided percutaneous coronary intervention in patients with serial stenoses within one coronary artery. *JACC Cardiovascular interventions*, 2012, vol. 5, 1013-8
- **GOTBERG M ; COOK CM ; SEN S ; NIJJER S ; ESCANED J ; DAVIES JE**. The Evolving Future of Instantaneous Wave-Free Ratio and Fractional Flow Reserve. *Journal of the American College of Cardiology.*, 2017, vol. 70, 1379-1402

- **GOSLING RD** ; **MORRIS PD** ; **LAWFORD PV** ; **HOSE DR** ; **GUNN JP**. Personalised Fractional Flow Reserve: Novel Concept to Optimise Myocardial Revascularization. *EuroIntervention : journal of EuroPCR in collaboration with the Working Group on Interventional Cardiology of the European Society of Cardiology*, 2018

- **COLLET C** ; **KATAGIRI Y** ; **MIYAZAKI Y** ; **ASANO T** ; **SONCK J** ; **VAN GEUNS RJ** ; **ANDREINI D** ; **BITTENCOURT MS** ; **KITSLAAR P** ; **TENEKECIOU-GLU E**. Impact of Coronary Remodeling on Fractional Flow Reserve.. *Circulation.*, 2018, vol. 137, 747-749
- **COLLET C** ; **ONUMA Y** ; **SONCK J** ; **ASANO T** ; **VANDELOO B** ; **KORNOWSKI R** ; **TU S** ; **WESTRA J** ; **HOLM NR** ; **XU B**. Diagnostic performance of angiography-derived fractional flow reserve: a systematic review and Bayesian meta-analysis. *European heart journal.*, 2018, vol. 39, 3314-3321